(19) Europäisches Patentamt
European Patent Office
Office européen des brevets

(11) Veröffentlichungsnummer : **0 384 889 B1**

(12) # EUROPÄISCHE PATENTSCHRIFT

(45) Veröffentlichungstag der Patentschrift : **13.10.93 Patentblatt 93/41**

(51) Int. Cl.$^5$ : **C07D 285/14,** C07D 417/06, A01N 43/82

(21) Anmeldenummer : **90810097.7**

(22) Anmeldetag : **13.02.90**

(54) **Mittel zum Schutz von Pflanzen gegen Krankheiten.**

(30) Priorität : **21.02.89 CH 617/89**

(43) Veröffentlichungstag der Anmeldung : **29.08.90 Patentblatt 90/35**

(45) Bekanntmachung des Hinweises auf die Patenterteilung : **13.10.93 Patentblatt 93/41**

(84) Benannte Vertragsstaaten : **AT BE CH DE DK ES FR GB GR IT LI LU NL SE**

(56) Entgegenhaltungen :
**EP-A- 0 313 512**
**GB-A- 1 176 799**

(73) Patentinhaber : **CIBA-GEIGY AG**
**Klybeckstrasse 141**
**CH-4002 Basel (CH)**

(72) Erfinder : **Kunz, Walter, Dr.**
**Buchenstrasse 9**
**CH-4104 Oberwil (CH)**
Erfinder : **Schurter, Rolf, Dr.**
**Holzmattstrasse 45**
**CH-4102 Binningen (CH)**

**EP 0 384 889 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue N-Acyl- und N-Sulfonyl-benz-1,2,3-thiadiazol-7-carbonsäureamide der nachstehenden Formel I. Die Erfindung betrifft ferner die Herstellung dieser Substanzen sowie die als Wirkstoff mindestens eine dieser Verbindungen enthaltenden Mittel. Die Erfindung betrifft darüber hinaus die Herstellung der genannten Mittel sowie die Verwendung der Wirkstoffe oder der Mittel zum Schutz von Pflanzen gegen den Befall durch schädliche Mikroorganismen, beispielsweise pflanzenschädigende Pilze, Bakterien und Viren.

Die erfindungsgemässen Verbindungen entsprechen der allgemeinen Formel I

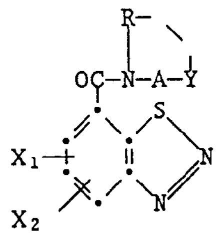

(I)

in welcher bedeuten:

$X_1$ und $X_2$ unabhängig voneinander Wasserstoff oder 1 bis 3 Halogen;

A Sulfonyl oder Carbonyl;

Y Phenyl oder mit $C_1$-$C_2$-Alkyl, $C_1$-$C_2$-Alkoxy, Halogen, $C_1$-$C_2$-Haloalkyl, $C_1$-$C_2$-Alkoxycarbonyl, $C_1$-$C_3$-Alkanoyloxymethyl, Cyano und/oder Nitro substituiertes Phenyl;

R Wasserstoff, $C_1$-$C_4$-Alkyl, $C_1$-$C_4$-Haloalkyl, $C_3$-$C_5$-Alkenyl oder $C_3$-$C_5$-Alkinyl;

und in welcher ferner die Gruppe

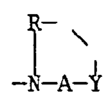

darstellt:

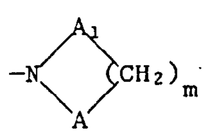, 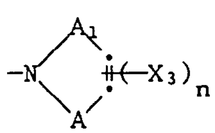 oder 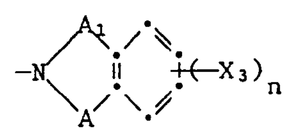,

worin bedeuten:

$A_1$ Methylen, Carbonyl oder Sulfonyl;

$X_3$ Wasserstoff, Methyl oder Halogen;

m 2, 3 oder 4; und

n Null, 1 oder 2.

Halogen bedeutet Fluor, Chlor, Brom oder Jod, bevorzugt Fluor und weiter in der Reihenfolge Chlor, Brom und Jod. Als Substituent in einzelnen Resten kann Halogen 1- bis 3-fach vertreten sein.

Unter Alkyl selbst oder als Bestandteil eines anderen Substituenten sind gerad- und verzweigtkettige Alkyle zu verstehen. Sie stellen je nach Zahl der angegebenen Kohlenstoffatome beispielsweise folgende Gruppen dar: Methyl, Ethyl sowie die Isomeren von Propyl oder Butyl, wie z.B. Isopropyl, Isobutyl, tert.-Butyl oder sek.-Butyl.

Alkenyl steht z.B. für Propenyl-(1), Allyl, Butenyl-(1), Butenyl-(2) oder Butenyl-(3) und Alkinyl bedeutet z.B. Propinyl-(2), Butinyl-(1) oder Pentinyl-(4).

Die Erfindung bezieht sich insbesondere auf Verbindungen der Formel I, in welcher bedeuten:

$X_1$ und $X_2$ unabhängig voneinander Wasserstoff oder 1 bis 3 Fluor;

A Sulfonyl oder Carbonyl;

Y mit Methyl, Methoxy, Halogen, Trifluormethyl, der $COOCH_3$-Gruppe, Cyano und/oder Nitro substituiertes

Phenyl;
R Wasserstoff, Methyl, Ethyl, Trifluormethyl, Allyl oder Propargyl; und in welcher die Gruppe

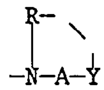

darstellt:

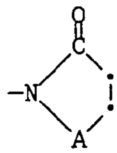

,

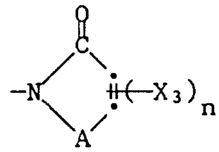

oder

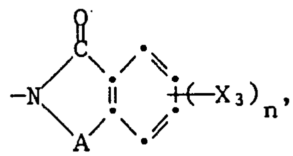

,

worin bedeuten:
A Sulfonyl oder Carbonyl;
$X_3$ Methyl oder Halogen; und
n Null, 1 oder 2.

Aufgrund ihrer besonderen pflanzenschützenden Eigenschaften lassen sich die Verbindungen der Formel I in folgende Gruppen einteilen:

1. Verbindungen der Formel I, worin bedeuten:
$X_1$ und $X_2$ unabhängig voneinander Wasserstoff oder Fluor;
A Sulfonyl oder Carbonyl;
Y mit Methyl, Methoxy, Halogen, Trifluormethyl und/oder der $COOCH_3$-Gruppe substituiertes Phenyl;
R Wasserstoff, Methyl, Ethyl, Trifluormethyl, Allyl oder Propargyl; und in welcher ferner die Gruppe

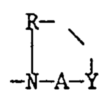

darstellt:

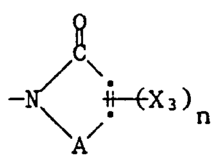

oder

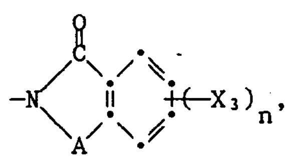

,

worin bedeuten:
A Sulfonyl oder Carbonyl;
$X_3$ Halogen; und
n Null oder 1.

2. Verbindungen der Formel I, worin bedeuten:
$X_1$ und $X_2$ unabhängig voneinander Wasserstoff oder Fluor;
A Sulfonyl;
Y mit Methyl, Methoxy, Fluor, Chlor, Trifluormethyl und/oder der $COOCH_3$-Gruppe substituiertes Phenyl;
R Wasserstoff, Methyl, Trifluormethyl, Allyl oder Propargyl; und in welcher ferner die Gruppe

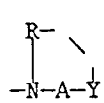

darstellt:

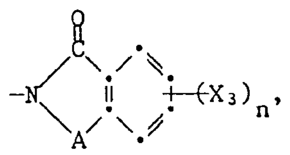

worin bedeuten:
A Sulfonyl;
$X_3$ Chlor; und
n Null oder 1.
3. Verbindungen der Formel I, worin bedeuten:
$X_1$ und $X_2$ unabhängig voneinander Wasserstoff oder Fluor;
A Sulfonyl;
Y mit Methyl, Fluor, Chlor und/oder Trifluormethyl substituiertes Phenyl;
R Wasserstoff, Methyl, Allyl oder Propargyl; und in welcher ferner für

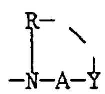

die Gruppe

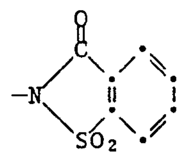

steht.

Die folgenden Verbindungen zeichnen sich durch besonders vorteilhafte pflanzenschützende Eigenschaften aus:
N-(Benz-1,2,3-thiadiazol-7-yl-carbonyl)-2-sulfobenzoesäureimid (Verb. Nr. 1.1);
N-4-Chlorphenylsulfonyl-benz-1,2,3-thiadiazol-7-carbonsäureamid (Verb. Nr. 1.4);
N-2-Fluorphenylsulfonyl-benz-1,2,3-thiadiazol-7-carbonsäureamid (Verb. Nr. 1.7);
N-Allyl-N-phenylsulfonyl-benz-1,2,3-thiadiazol-7-carbonsäureamid (Verb. Nr. 1.28);
N-Phenylsulfonyl-4-fluor-benz-1,2,3-thiadiazol-7-carbonsäureamid (Verb. Nr. 2.1);
N-4-Methylphenylsulfonyl-5-fluor-benz-1,2,3-thiadiazol-7-carbonsäureamid (Verb. Nr. 2.21);
N-Phenylsulfonyl-benz-1,2,3-thiadiazol-7-carbonsäureamid (Verb. Nr. 4.1);
N-(5-Fluor-benz-1,2,3-thiadiazol-7-carbonyl)-2-sulfobenzoesäureimid (Verb. Nr. 4.2).

Es wurde nun überraschenderweise gefunden, dass die erfindungsgemässen Verbindungen der Formel I durch ihre Verwendung den Befall von Pflanzen durch schädliche Mikroorganismen verhindern und damit den befallsbedingten Schädigungen der Pflanzen vorbeugen. Für die erfindungsgemässen Wirkstoffe ist charakteristisch, dass der Schutz der Pflanzen sowohl durch direkte Einwirkung auf die pflanzenschädigenden Mikroorganismen mittels Blattapplikation oder Bodenapplikation als auch durch Aktivierung und Stimulierung des pflanzeneigenen Abwehrsystems (Immunisierung) eintreten kann. Der grosse Vorteil der Verbindungen der Formel I besteht darin, dass die Gesunderhaltung der mit diesen Stoffen behandelten Pflanzen auch aus eigener Kraft ohne Einsatz weiterer mikrobizider Substanzen während der Vegetationsperiode gewährleistet werden kann. Demzufolge ist es möglich, durch die Anwendung der erfindungsgemässen Wirkstoffe nachteilige Nebeneffekte, wie sie bei der direkten Parasitenbekämpfung mit chemischen Substanzen z.B. einerseits durch Schädigung der Nutzpflanzen (Phytotoxizität) und andererseits durch Hervorrufung von Resistenzerscheinungen bei den schädlichen Mikroorganismen in Erscheinung treten können, zu vermeiden, was vorteilhafterweise ein völlig ungestörtes Wachstum der Nutzpflanzen zur Folge hat.

Aufgrund der doppelten Wirkungsweise der erfindungsgemässen Verbindungen der Formel I, nämlich einerseits der direkten Bekämpfung der Pflanzenpathogene und andererseits der Erhöhung der allgemeinen Abwehrbereitschaft der mit diesen Wirkstoffen behandelten Pflanzen durch Immunisierung kann ein breit gefächerter Schutz der Pflanzen gegen Krankheiten erzielt werden. Die Anwendung der erfindungsgemässen Wirkstoffe ist deshalb besonders für praktische Bedingungen geeignet. Darüber hinaus bewirkt die den Verbindungen der Formel I eigene systemische Aktivität, dass sich der Schutzeffekt auch auf zuwachsende Pflanzenteile der behandelten Pflanzen erstreckt.

Die allgemein pflanzenschützende Aktivität der erfindungsgemässen Wirkstoffe ist z.B. gegen die den folgenden Klassen angehörenden phytopathogenen Fungi wirksam: Fungi imperfecti (z.B. Botrytis, Helminthosporium, Fusarium, Septoria, Cercospora und Alternaria); Basidiomyceten (z.B. die Gattungen Hemileia, Rhizocotonia, Puccinia); Ascomyceten (z.B. Venturia, Podosphaera, Erysiphe, Monilinia, Uncinula).

Darüber hinaus können die Wirkstoffe besonders vorteilhaft gegen folgende Schadorganismen eingesetzt werden:

Pilze, wie z.B. Oomyceten (z.B. Plasmopara viticola, Phytophthora infestans, Peronospora tabacina, Pseudoperonospora, Bremia letucae), Fungi imperfecti (z.B. Colletotrichum lagenarium, Piricularia oryzae, Cercospora nicotinae), Ascomyceten (z.B. Venturia inaequalis);

Bakterien, wie z.B. Pseudomonaden (Pseudomonas lachrymans, Pseudomonas tomato, Pseudomonas tabaci); Xanthomonaden (z.B. Xanthomonas oryzae, Xanthomonas vesicatoria); Erwinia (z.B. Erwinia amylovora); und Viren, wie z.B. das Tabakmosaikvirus.

Die erfindungsgemässen Verbindungen können zum Schutz von Pflanzen unterschiedlicher Nutzkulturen eingesetzt werden.

Für den Einsatz der erfindungsgemässen Verbindungen der Formel I im Rahmen der Erfindung sind beispielsweise folgende Pflanzenarten geeignet: Getreide (Weizen, Gerste, Roggen, Hafer, Reis, Sorghum und Verwandte); Rüben (Zucker- und Futterrüben); Kern-, Stein- und Beerenobst (Aepfel, Birnen, Pflaumen, Pfirsiche, Mandeln, Kirschen, Erdbeeren, Himbeeren und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Grapefruit, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen). Diese Aufzählung stellt keine Limitierung dar.

Als besonders geeignete Zielkulturen für die Anwendung des erfindungsgemässen Verfahrens sind folgende Pflanzen anzusehen: Gurken, Tabak, Reben, Reis, Pfeffer, Kartoffeln, Tomaten, Weizen, Gerste, Birnen und Aepfel.

Die Verbindungen der Formel I werden hergestellt, indem man umsetzt: eine Benzthiadiazolverbindung der Formel II

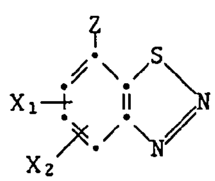

(II)

mit einer Amidverbindung der Formel III

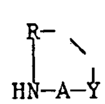

(III)

in Gegenwart einer Base und gegebenenfalls mit einem Katalysator, wie z.B. 4-Dialkylaminopyridin, speziell 4-Dimethylaminopyridin, in inerten Lösungsmitteln, wobei Z die Reste COOH, HalCO, $COOC_1$-$C_5$-Alkyl,

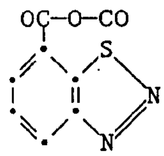

oder

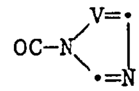

bedeutet, V CH oder H und Hal Halogen darstellen und $X_1$, $X_2$, A, Y und R die unter der Formel I angegebenen Bedeutungen besitzen. Die Reaktion findet bei Temperaturen von -10° bis 200°C, bevorzugt von 0° bis 100°C, statt.

Nach einem weiteren Verfahren zur Herstellung der Verbindungen der Formel I werden folgende Reaktionen durchgeführt:
eine Verbindung der Formel II

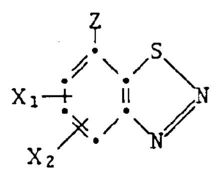

(II)

wird zunächst durch Reaktion mit einer Aminoverbindung der Formel IV

$H_2N$-R (IV)

in Gegenwart einer Base in inerten Lösungsmitteln in eine Amidverbindung der Formel V

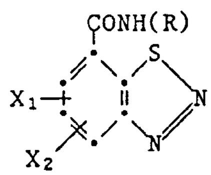

(V)

überführt und diese anschliessend mit einem Säurederivat der Formel VI

Z'-A-Y (VI)

in Gegenwart einer Base in inerten Lösungsmitteln umgesetzt, wobei Z' die Reste OH, Hal, $OC_1$-$C_5$-Alkyl, oder

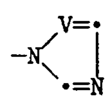

bedeutet,
oder der Rest Z'-A für

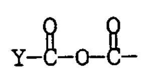

steht, V CH oder N und Hal Halogen darstellen und $X_1$, $X_2$, A, Y und R die unter der Formel I angegebenen Bedeutungen besitzen. Die Reaktionen finden bei Temperaturen von -10° bis 200°C, bevorzugt von 0° bis 100°C, statt.

Verbindungen der Formel II, in denen Z COOH bedeutet, können entweder wie in der Literatur beschrieben (vgl. J.Chem.Soc. 1971, 3997) oder vorteilhafterweise gemäss den nachstehend angegebenen Herstellungsbeispielen erhalten werden. Säurehalogenide, die unter die Formel II fallen, werden aus den entsprechenden freien Carbonsäuren z.B. mit Thionylchlorid, Phosgen, Oxalylchlorid oder 1-Chlor-N,N-2-trimethylpropenylamin (vgl. L. Ghosez, J.Chem.Soc. Comm. 1979, 1180) hergestellt. Säureanhydride, die unter die Formel II fallen, können z.B. durch Erwärmen der entsprechenden freien Säure mit Acetanhydrid erhalten

werden. Imidazolide und Triazolide, die unter die Formel II fallen, werden aus den Carbonsäuren durch Umsetzung mit N,N-Carbonyldiimidazol oder N,N-Carbonylditriazol gewonnen (vgl. H.A. Staab, Angew. Chemie 1964, 132).

Als Basen kommen organische und anorganische in Betracht, z.B. tertiäre Amine wie Trialkylamine (Trimethylamin, Triethylamin, Tripropylamin usw.), Pyridinbasen (Pyridin, 4-Dimethylaminopyridin, 4-Pyrrolidylaminopyridin, Collidin), Oxide und Hydroxide, Carbonate und Hydrogencarbonate von Alkali- und Erdalkalimetallen sowie Hydride, wie z.B. Natriumhydrid oder Calciumhydrid, oder Alkyllithium-Verbindungen, wie z.B. n-Butyllithium.

Als Reaktionsmedien in Anpassung an die jeweiligen Reaktionsbedingungen werden geeignete reaktionsinerte Lösungs- und Verdünnungsmittel verwendet. Als Beispiele sind zu nennen: aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Ketone wie Aceton, Diethylketon, Methylethylketon; sowie Gemische solcher Lösungsmittel untereinander.

Aminoverbindungen der Formel III sind bekannt oder lassen sich nach dem Fachmann bekannten Methoden herstellen.

Verbindungen der Formel V, in denen R Wasserstoff bedeutet, können hergestellt werden, indem man Carbonsäureester, die unter die Formel II fallen, vorzugsweise der Methylester, mit 1 bis 100 Aequivalenten, bevorzugt 1 bis 30 Aequivalenten, flüssigem Ammoniak in einem Autoklaven bei $1 \cdot 10^5$ Pa bis $100 \cdot 10^5$ Pa, vorzugsweise bei $5 \cdot 10^5$ Pa bis $60 \cdot 10^5$ Pa, bei 0° bis 160°C, vorzugsweise 20° bis 120°C, in inerten Lösungsmitteln umsetzt.

Verbindungen der Formel V, in denen R Wasserstoff bedeutet, können ferner hergestellt werden, indem man Säurehalogenide, die unter die Formel II fallen, mit Hexamethyldisilazan der Formel $[(CH_3)_2Si]_2NH$ oder einem analogen Silylamin in inerten Lösungsmitteln bei Temperaturen von -10° bis 80°C, bevorzugt 0° bis 40°C umsetzt und anschliessend hydrolytisch z.B. mit einem Alkohol wie Methanol und verdünnter Mineralsäure, z.B. Schwefelsäure behandelt (vgl. Synthetic Communications 1985, 519).

Die im Rahmen der Erfindung zur Anwendung gelangenden mikrobiziden Mittel zum Schutz von Pflanzen gegen Krankheiten, welche die Verbindungen der Formel I als Aktivstoffe enthalten, sind als Teil der Erfindung zu betrachten.

Wirkstoffe der Formel I werden üblicherweise in Form von Zusammensetzungen verwendet und können gleichzeitig oder nacheinander mit weiteren Wirkstoffen auf die Pflanze oder deren Umgebung gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein Verfahren zur Anwendung eines Wirkstoffes der Formel I bzw. eines agrochemischen Mittels, das mindestens einen dieser Wirkstoffe enthält, ist das Aufbringen auf die Pflanze (Blattapplikation). Die Wirkstoffe der Formel I können aber auch über den Erdboden durch das Wurzelwerk in die Pflanze gelangen (Bodenapplikation), indem man den Standort der Pflanze mit einer flüssigen Zubereitung tränkt oder die Substanzen in fester Form in den Boden einbringt z.B. in Form von Granulat. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet (Beizapplikation). Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt. Zu diesem Zweck werden sie z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt bei 100 g bis 2 kg AS/ha, insbesondere bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden hergestellt durch inniges Vermischen und/oder Vermahlen der Wirkstoffe mit Streckmitteln, wie z.B. mit Lösungsmitteln, festen Trägerstoffen, und gegebenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid; sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I nicht-ionogene, kation- und/oder anionaktive Tenside mit guten Emulgier-, Dispergier- und Netzeigenschaften in Betracht. Unter Tensiden sind auch Tensidgemische zu verstehen.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedere, gegebenenfalls halogenierte Alkyl-, Benzyl- oder niedere Hydroxyalkylreste aufweisen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure oder von natürlichen Fettsäuregemischen, die z.B. aus Kokosnuss- oder Talgöl gewonnen werden können, genannt.

Als synthetische Tenside können insbesondere Fettalkoholsulfonate, Fettalkoholsulfate, sulfonierte Benzimidazolderivate oder Alkylsulfonate Verwendung finden. Die Fettalkoholsulfonate oder -sulfate liegen in der Regel als Alkali-, Erdalkali- oder gegebenenfalls substituierte Ammoniumsalze vor und weisen einen Alkalirest mit 8 bis 22 C-Atomen auf.

Als nicht-ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Die nachfolgenden Beispiele dienen zur näheren Erläuterung der Erfindung, ohne dieselbe einzuschränken.

1. Herstellungbeispiele

1. Herstellung von N-(Phenylsulfonyl)-benz-1,2,3-thiadiazol-7-carbonsäureamid

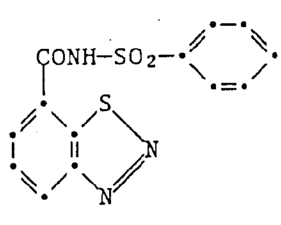

3,2 g Benzolsulfonamid werden in 50 ml abs. Pyridin bei 0 - 5°C vorgelegt und unter Rühren bei dieser

Temperatur tropfenweise mit einer Lösung von 3.9 g Benz-1,2,3-thiadiazol-7-carbonsäurechlorid in 20 ml Methylenchlorid versetzt. Nach Rühren über Nacht wird auf Eiswasser gegossen, mit Salzsäure leicht sauer gestellt, mit Methylenchlorid extrahiert, die Extrakte mit Wasser gewaschen, getrocknet und eingedampft. Der Rückstand wird in Tetrahydrofuran warm gelöst, mit etwas Kieselgel behandelt, filtriert, eingedampft und dann aus Tetrahydrofuran/Hexan umkristallisiert. Die anfallende Titelverbindung schmilzt bei 231 - 233° C.

2.Herstellung von N-(Benz-1,2,3-thiadiazol-7-yl)-carbonyl-2-sulfobenzoesäureimid

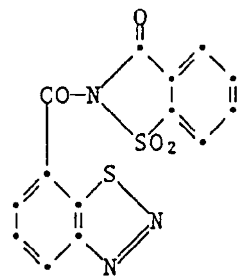

Eine Suspension von 4 g des Natriumsalzes von Saccharin in 60 ml Methylenchlorid wird nach Zugabe einer Spatelspitze 4-Dimethylaminopyridin bei max. 20°C tropfenweise mit einer Lösung von 3.9 g Benz-1,2,3-thiadiazol-7-carbonsäurchlorid in 20 ml Methylenchlorid versetzt. Ueber Nacht wird bei Raumtemperatur gerührt, die Suspension am nächsten Tag auf Eiswasser gegossen und mit Essigsäureethylester extrahiert. Die Extrakte werden mit verdünnter Natriumhydrogencarbonat-Lösung und Wasser gewaschen, getrocknet und eingedampft. Der Rückstand liefert nach Umkristallisation aus Tetrahydrofuran/Hexan die Titelverbindung mit einem Smp. von 173 - 175°C.

3. Herstellung von Benz-1,2,3-thiadiazol-7-carbonsäureamid (Zwischenprodukt)

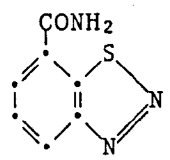

In einem Autoklaven werden zu einer Lösung von 19.4 g Benz-1,2,3-thiadiazol-7-carbonsäuremethylester in 70 ml Tetrahydrofuran bei Raumtemperatur 17 g Ammoniak unter Druck aufgepresst. Anschliessend wird aufgeheizt und während ca. 20 Std. bei 80 - 90°C gehalten, wobei sich ein Innendruck von max. 55·105 Pa aufbaut. Anschliessend wird partiell eingedampft, der entstandene Niederschlag abfiltriert, mit kaltem Tetrahydrofuran gewaschen und getrocknet. Es resultierten 14.9 g beige Kristalle mit einem Smp. von 270 - 272°C.

4. Herstellung von N-(Benz-1,2,3-thiadiazol-7-carbonyl)-pyrrolidon-2

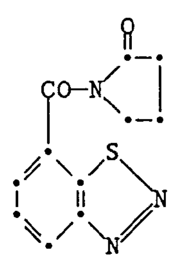

Unter Stickstoffatmosphäre und Rühren wird eine bei 10°C vorgelegte Lösung von 0.348 ml 2-Pyrrolidon und 0.653 ml Triethylamin in 10 ml Tetrahydrofuran bei maximal 15°C tropfenweise mit 0.576 ml Trimethylchlorsilan versetzt. Dann wird während 1 Stunde gegen Raumtemperatur gerührt und schliesslich unter erneuter Eiswasser-Kühlung innerhalb 10 Min. bei maximal 20°C eine Lösung von 0.9 g Benz-1,2,3-thiadiazol-7-carbonsäurechlorid in 5 ml Tetrahydrofuran zugetropft. Ueber Nacht wird bei Raumtemperatur gerührt, mit Eiswasser und Essigsäureethylester versetzt, die organische Phase abgetrennt und die wässrige Phase noch zweimal mit Essigsäureethylester extrahiert. Die vereinigten Extrakte werden mit Hydrogencarbonat-Lösung und Wasser gewaschen, über Natriumsulfat getrocknet und über wenig Kieslgel filtriert. Das Filtrat wird eingedampft und der Rückstand aus Tetrahydrofuran-Hexan umgelöst. Es resultieren 0.7 g der Titelverbindung vom Smp. 146-147°C.

5.Herstellung des symmetrischen Anhydrids der 1,2,3-Benzthiadiazol-7-carbonsäure (Zwischenprodukt)

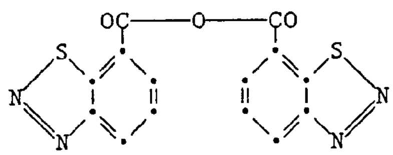

3 g 1,2,3-Benzthiadiazol-7-carbonsäure werden in 50 ml Acetanhydrid während 24 Stunden unter Rückfluss gekocht. Dann wird die dünne Suspension unter Vakuum eingedampft, der feste Rückstand mit Ether aufgeschlämmt und abfiltriert. Es resultieren 4,3 g Anhydrid vom Smp. 117-119°C. Die gleiche Verbindung wird z.B. auch durch Erwärmen der Carbonsäure mit Bis-(2-oxo-3-oxazolidinyl)-phosphin-säurechlorid in trockenem Tetrahydrofuran erhalten (vgl. Synthesis 1981, 616).

6. Herstellung von 7-Methoxycarbonyl-benzo-1,2,3-thiadiazol (Zwischenprodukt

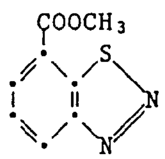

a) 100 g (0,35 Mol) 2-Benzylthio-3,5-diaminobenzoesäuremethylester werden portionsweise zu 250 ml konz. Salzsäure und 110 ml Wasser gegeben und 1,5 Stunden bei Raumtemperatur gerührt. Dann wird das Gemisch auf -5°C gekühlt und während 2,5 Stunden unter Rühren eine Lösung von 48,5 g (0,70 Mol) Natriumnitrit in 210 ml Wasser zugetropft. Der Rührvorgang wird weitere 2 Stunden bei 0°C fortgesetzt. Anschliessend werden 190 ml 50 %ige unterphosphorige Säure während 2 1/2 Stunden zugetropft. Darauf lässt man die Temperatur während 19 Stunden auf 20°C steigen. Das erhaltene Produkt wird abfiltriert, mit Wasser gewaschen und getrocknet. Zur Reinigung wird das Produkt in Essigsäureethylester/Methylenchlorid gelöst, durch Kieselgel filtriert, evaporiert und durch Zugabe von Hexan kristallisiert. Ausbeute: 44,4 g (65 % d.Th.); Smp. 132°C.

b) 576 g (2 Mol) 3,5-Diamino-2-benzylthio-benzoesäure-methylester werden in 500 ml 1,4-Dioxan gelöst und unter Rühren und Kühlen bei 0° bis 5°C zu vorgelegter 5N Salzsäure (3 l) getropft. Anschliessend wird die feine Suspension auf -17° bis -20°C abgekühlt und innerhalb 1,25 Stunden unter Niveau tropfenweise mit 294 g Natriumnitrit in 500 ml Waser versetzt. Unter weiterem Rühren wird die Innentemperatur innerhalb 1 Stunde auf -5°C ansteigen gelassen und während 2 Stunden gehalten. Dann wird auf -15°C abgekühlt und die Suspension unter Rühren portionenweise in auf -10° bis -15°C gekühlte unterphosphorige Säure (1,1 l) eingetragen, wobei Stickstoff entweicht. Nach beendeter Zugabe wird die Innentemperatur innerhalb 5-6 Stunden auf Raumtemperatur ansteigen gelassen, der gebildete Niederschlag abfiltriert, diese mit 2,5 l Methylenchlorid verrührt, wieder vom nichtgelösten Teil abfiltriert und das Filtrat vom Wasser getrennt. Anschliessend wird die organische Phase über Natriumsulfat getrocknet, mit 300 g Kieselgel verrührt, erneut filtriert, mit Methylenchlorid nachgewaschen und das Filtrat eingedampft. Aus Methanol umkristallisiert resultieren insgesamt 244,8 g (63,1 % d. Th.) beige Kristalle vom Smp. 130°-133°C.

Tabelle 1

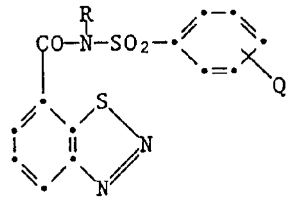

| Verb. Nr. | Q | R | physikalische Daten |
|---|---|---|---|
| 1.1 | H | H | Smp. 231-233°C |
| 1.2 | 2,3-di-Cl | H(Pyridiniumsalz) | Smp. 256-259°C |
| 1.3 | H | $CH_3$ | |
| 1.4 | 4-Cl | H | Smp. 250-252°C |
| 1.5 | 2,4-di-Cl | H | Smp. 279-280°C |
| 1.6 | 2,4,6-tri-Cl | H | Smp. 248-249°C |
| 1.7 | 2-F | H | Smp. 270-272°C |
| 1.8 | 4-F | H | |
| 1.9 | $2-CH_3-4-Cl$ | H | |
| 1.10 | 2,6-Di-Cl | H | |
| 1.11 | $3-CF_3$ | H | |
| 1.12 | $3-NO_2-4-Cl$ | H | |
| 1.13 | 3-Cl | H | |
| 1.14 | $2-CH_3$ | H | |
| 1.15 | $2-CH_2OCOCH_3$ | H | |
| 1.16 | $2-COOC_2H_5$ | H | |
| 1.17 | 2-F | Me | |
| 1.18 | 4-F | $CH_2-CH=CH_2$ | |
| 1.19 | 2,4-Di-F | H | |
| 1.20 | $3-NO_2$ | H | Smp. 273-274°C |
| 1.21 | 3-Cl | $C_2H_5$ | |
| 1.22 | $3-CF_3$ | $n-C_4H_9$ | |
| 1.23 | H | $C_2H_5$ | |
| 1.24 | H | $C_3H_7$ | |
| 1.25 | $4-CH_3$ | H | |
| 1.26 | $4-CH_3$ | $CH_3$ | |
| 1.27 | $2-CF_3-4-Cl$ | H | |
| 1.28 | H | $CH_2-CH=CH_2$ | Smp. 100-102°C |
| 1.29 | H | $CH_2-C\equiv CH$ | |
| 1.30 | H | $-CH_2CH=CH-CH_3$ | |
| 1.31 | H | $-CH_2C\equiv C-CH_3$ | |
| 1.32 | H | $-CH_2CH=CH-C_2H_5$ | |
| 1.33 | 3-CN | H | |
| 1.34 | $2-COOCH_3$ | $CH_3$ | |
| 1.35 | $2-CH_2OC_3H_7-n$ | H | |
| 1.36 | $4-OCH_3$ | H | Smp. 230-231°C |
| 1.37 | $4-OC_2H_5$ | H | |
| 1.38 | $2-C_2H_5$ | H | |
| 1.39 | $CH_2CCl_3$ | H | |

Tabelle 2

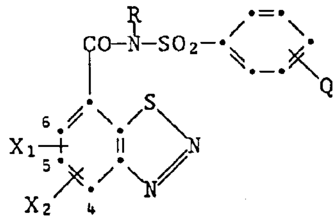

| Verb. Nr. | Q | $X_1$ | $X_2$ | R | physikalische Daten |
|---|---|---|---|---|---|
| 2.1 | H | 4-F | | H | |
| 2.2 | H | 6-F | | H | Smp. 157-158°C |
| 2.3 | H | 5-F | 4-F | H | |
| 2.4 | H | 5-F | H | $CH_2$-CH=$CH_2$ | |
| 2.5 | H | 5-F | H | H | |
| 2.6 | H | 5-Cl | H | H | |
| 2.7 | H | 5-Br | H | H | |
| 2.8 | H | 5-J | H | H | |
| 2.9 | 2,3-di-Cl | 5-J | H | H | |
| 2.10 | 2,3-di-Cl | 5-F | H | H *) | Smp. 218-220°C |
| 2.11 | 4-O-$CH_3$ | 5-F | H | H | Smp. 196-198°C |
| 2.12 | 4-F | 5-F | H | H | |
| 2.13 | 3-F | 6-F | H | H | |
| 2.14 | 2,6-di-Cl | 5-F | 6-F | H | |
| 2.15 | 2,3-di-Cl | 5-Br | H | $CH_3$ | |
| 2.16 | 4-O-$C_2H_5$ | 5-F | H | $CH_3$ | |
| 2.17 | 2-$CH_2$Cl | 5-F | H | $CH_3$ | |
| 2.18 | 2-$CH_2$OC(O)$CH_3$ | 5-F | H | $CH_3$ | |
| 2.19 | 4-$CH_3$ | 5-F | H | $CH_3$ | |
| 2.20 | 4-Cl | 5-F | H | H | Smp. 165-167°C |
| 2.21 | 4-$CH_3$ | 5-F | H | H | Smp. 231-233°C |
| 2.22 | 4-F | 6-F | H | H | Smp. 207-208°C |

*) (Pyridiniumsalz)

Tabelle 3

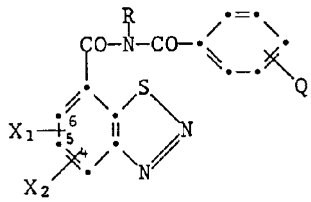

| Verb. Nr. | Q | $X_1$ | $X_2$ | R | physikalische Daten |
|---|---|---|---|---|---|
| 3.1 | H | H | H | H | |
| 3.2 | $4-CH_3$ | 5-F | H | H | |
| 3.3 | 4-Cl | 6-F | H | H | |
| 3.4 | $4-OC_2H_5$ | H | H | H | |
| 3.5 | H | H | H | $CH_3$ | |
| 3.6 | H | H | H | $CH_2-CH=CH_2$ | |
| 3.7 | H | H | H | $CH_2-C\equiv CH$ | |
| 3.8 | 2,6-di-Cl | 5-F | H | $CH_2-CH=CH_2$ | |
| 3.9 | 2,6-di-Cl | 5-F | H | $C_2H_5$ | |
| 3.10 | H | 5-F | 6-F | H | |
| 3.11 | $3-CF_3$ | H | H | H | |
| 3.12 | $3-NO_2$ | H | H | H | |
| 3.13 | 2-F | H | H | H | |
| 3.14 | $3-CF_3$ | 5-Cl | H | H | |
| 3.15 | H | 5-Br | H | H | |
| 3.16 | $2-CH_3$ | H | H | H | |
| 3.17 | 2,4,6-tri-Cl | H | H | H | |
| 3.18 | 2,3-di-Cl | H | H | H | |
| 3.19 | $2-COO-CH_3$ | H | H | $CH_3$ | |
| 3.20 | H | 5-J | H | $CH_3$ | |

Tabelle 4

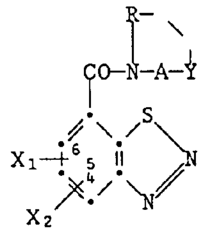

| Verb. Nr. | 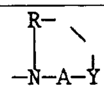 | $X_1$ | $X_2$ | physikalische Daten |
|---|---|---|---|---|
| 4.1 | 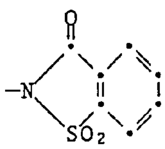 | H | H | Smp. 173-175°C |
| 4.2 | 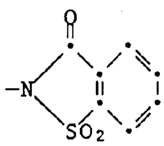 | 5-F | H | Smp. 177-179°C |
| 4.3 | 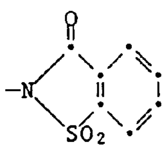 | 5-Cl | H | |
| 4.4 | 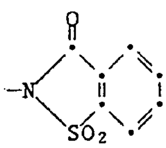 | 5-F | 6-F | |
| 4.5 | 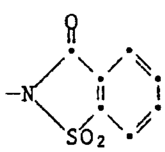 | 5-Br | H | |
| 4.6 | 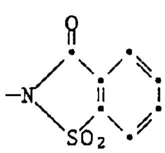 | 4-F | H | |
| 4.7 | 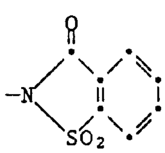 | 5-J | H | |

| Verb. Nr. | 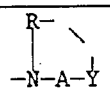 | $X_1$ | $X_2$ | physikalische Daten |
|---|---|---|---|---|
| 4.8 | 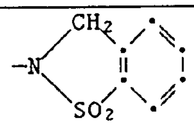 | H | H | |
| 4.9 | 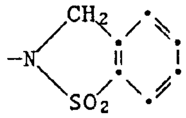 | 5-F | H | |
| 4.10 | 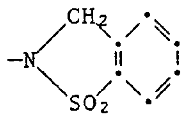 | 5-J | H | |
| 4.11 | 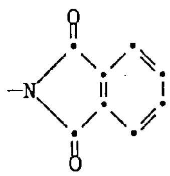 | H | H | Smp. 259-260°C |
| 4.12 | 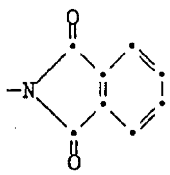 | 5-F | H | Smp. 222-223°C |
| 4.13 | 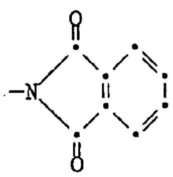 | 5-Cl | H | |
| 4.14 | 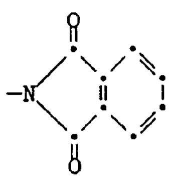 | 5-Br | H | |
| 4.15 | 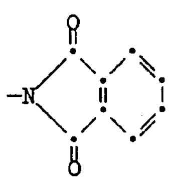 | 6-F | H | |
| 4.16 | 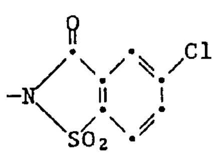 | H | H | |

| Verb. Nr. | 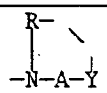 | $X_1$ | $X_2$ | physikalische Daten |
|---|---|---|---|---|
| 4.17 | 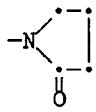 | H | H | |
| 4.18 | 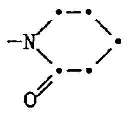 | H | H | |
| 4.19 | 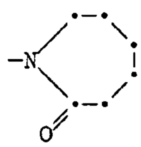 | H | H | |
| 4.20 | 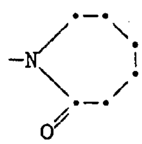 | 5-F | H | |
| 4.21 | 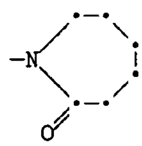 | 6-F | H | |
| 4.22 | 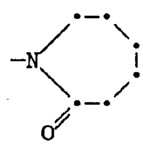 | 5-Br | H | |
| 4.23 | 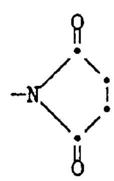 | H | H | Smp. 202-203°C |
| 4.24 | 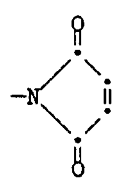 | H | H | |
| 4.25 | 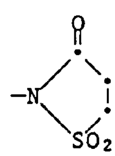 | H | H | |

| Verb. Nr. | 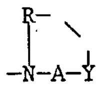 | $X_1$ | $X_2$ | physikalische Daten |
|---|---|---|---|---|
| 4.26 | 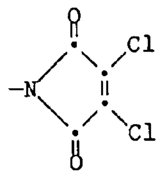 | H | H | |
| 4.27 | 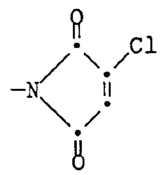 | 5-F | H | |
| 4.28 | 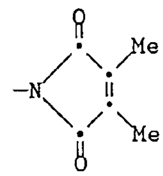 | H | H | |
| 4.29 | 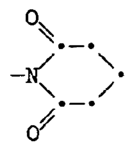 | H | H | |

2. Formulierungsbeispiele für Wirkstoffe der Formel I (% = Gewichtsprozent)

| 2.1 Spritzpulver | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle homogen vermahlen. Man erhält Spritzpulver, die sich mit Wasser zu Suspensionen jeder gewünschten Konzentration verdünnen lassen.

| 2.2 Emulsions-Konzentrat | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 2.3 Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

| 2.4 Extruder Granulat | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.5 Umhüllungs-Granulat | |
|---|---|
| Wirkstoff aus den Tabellen | 3 % |
| Polyethylenglykol (MG 200) | 3 % |
| Kaolin | 94 % |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.6 Suspensions-Konzentrat

| | | |
|---|---|---|
| Wirkstoff aus den Tabellen | 40 | % |
| Ethylenglykol | 10 | % |
| Nonylphenolpolyethylenglykolether (15 Mol Ethylenoxid) | 6 | % |
| N-Lingninsulfonat | 10 | % |
| Carboxymethylcellulose | 1 | % |
| 37%ige wässrige Formaldehyd-Lösung | 0,2 | % |
| Silikonöl in Form einer 75%igen wässrigen Emulsion | 0,8 | % |
| Wasser | 32 | % |

Der fein gemahlene Wirkstoff wird mit den Zusatzstoffen innig vermischt. Man erhält ein Suspensions-Konzentrat, aus welchem durch Verdünnen mit Wasser Suspensionen jeder gewünschten Konzentration hergestellt werden können.

3. Biologische Beispiele

Beispiel 3.1: Wirkung gegen Colletotrichum lagenarium auf Cucumis sativus L.

a) Gurkenpflanzen werden nach 2-wöchiger Anzucht mit einer aus Spritzpulverdes Wirkstoffes hergestellten Spritzbrühe besprüht (Konzentration: 200 ppm). Nach 48 Stunden werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23°C inkubiert. Die Inkubation wird dann bei normaler Luftfeuchtigkeit und bei 22°C bis 23°C weitergeführt.

Die Beurteilung der Schutzwirkung erfolgt aufgrund des Pilzbefalls 7-8 Tage nach der Infektion.

b) Gurkenpflanzen werden nach 2-wöchiger Anzucht mit einer aus Spritzpulver des Wirkstoffes hergestellten Spritzbrühe durch Bodenapplikation behandelt (Konzentration: 60 oder 20 ppm bezogen auf das Bodenvolumen). Nach 48 Stunden werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23°C inkubiert. Die Inkubation wird dann bei normaler Luftfeuchtigkeit und bei 22°C weitergeführt.

Die Beurteilung der Schutzwirkung erfolgt aufgrund des Pilzbefalls 7-8 Tage nach der Infektion.

c) Gurkenpflanzen werden nach 2-wöchiger Anzucht mit einer aus Spritzpulver des Wirkstoffes hergestellten Spritzbrühe besprüht (Konzentration: 200 ppm).

Nach 3 Wochen werden die Pflanzen mit einer Sporensuspension $1.5 \cdot 10^5$ Sporen/ml) des Pilzes infiziert und für 36 Stunden bei hoher Luftfeuchtigkeit und einer Temperatur von 23°C inkubiert. Die Inkubation wird dann bei normaler Luftfeuchigkeit und bei 22° bis 23°C weitergeführt.

Die Beurteilung der Schutzwirkung erfolgt aufgrund des Pilzbefalls 7-8 Tage nach der Infektion.

Verbindungen aus den Tabellen 1 bis 4 zeigten in den Tests (a) und (b) gute Wirkung. So reduzierte z.B. die Verbindung 4.1 den Pilzbefall auf 0 bis 20 %. Unbehandelte jedoch infizierte Kontrollpflanzen wiesen dagegen einen Colletotrichum-Befall von 100 % auf.

Beispiel 3.2: Wirkung gegen Phytophthora infestans auf Tomatenpflanzen

a) Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20°C.

b) Zu Tomatenpflanzen wird nach 3-wöchiger Anzucht eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung

des Pilzbefalls erfolgt nach einer Inkubation der infizierten Pflanzen während 5 Tagen bei 90-100 % relativer Luftfeuchtigkeit und 20°C.

Verbindungen aus den Tabellen 1 bis 4 zeigten gegen den Phytophthora-Pilz eine gute Schutzwirkung. So reduzierten z.B. die Verbindungen 1.1, 1.2 und 4.1 den Pilzbefall auf 0 bis 20 %. Unbehandelte jedoch infizierte Kontrollpflanzen wiesen dagegen einen 100 %igen Phytophthora-Befall auf.

Beispiel 3.3: Wirkung gegen Plasmopara viticola auf Reben

a) Im 4-5 Blattstadium werden Rebensämlinge mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20°C wird der Pilzbefall beurteilt.

b) Im 4-5 Blattstadium werden Rebensämlinge mit einer Sporangiensuspension des Pilzes infiziert. Nach einer Inkubation während 24 Stunden in einer Feuchtkammer bei 95-100 % relativer Luftfeuchtigkeit und 20°C werden die infizierten Pflanzen getrocknet und mit einer aus Spritzpulver des Wirkstoffes hergestellten Spritzbrühe (0,06 % Aktivsubstanz) besprüht. Nach dem Antrocknen des Spritzbelages werden die behandelten Pflanzen wieder in die Feuchtkammer gebracht. Die Beurteilung des Pilzbefalls erfolgt 6 Tage nach der Infektion.

Verbindungen aus den Tabellen 1 bis 4 zeigten gegen Plasmopara viticola eine gute Schutzwirkung (Befall 0-20 %), so z.B. 1.20. Unbehandelte jedoch infizierte Kontrollpflanzen wiesen dagegen einen 100%igen Plasmopara-Befall auf.

Beispiel 3.5: Wirkung gegen Piricularia oryzae auf Reispflanzen

a) Reispflanzen werden nach 2-wöchiger Anzucht mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach 5 Tagen Inkubation bei 95-100 % relativer Luftfeuchtigkeit und 24°C wird der Pilzbefall beurteilt.

b) Zu 2-wöchigen Reispflanzen wird eine aus Spritzpulver des Wirkstoffes hergestellte Spritzbrühe gegossen (0,006 % Aktivsubstanz bezogen auf das Erdvolumen). Darauf werden die Töpfe mit Wasser soweit gefüllt, dass die untersten Stengelteile der Reispflanzen im Wasser stehen. Nach 96 Stunden werden die behandelten Reispflanzen mit einer Konidiensuspension des Pilzes infiziert. Nach einer Inkubation der infizierten Pflanzen während 5 Tagen bei 95 - 100 % relativer Luftfeuchtigkeit und ca. 24°C wird der Pilzbefall beurteilt.

Reispflanzen, die mit einer Spritzbrühe behandelt wurden, die als Aktivsubstanz eine Verbindung aus den Tabellen 1 bis 4 enthielt, zeigten im Vergleich zu unbehandelten Kontrollpflanzen (100 % Befall) nur geringen Pilzbefall. So reduzierten z.B. im Test (a) die Verbindungen 1.1, 1.2 und im Test b) die Verbindungen 1.1, 1.2 und 4.1 den Befall auf 0 bis 20 %.

Beispiel 3.6: Wirkung gegen Xanthomonas oryzae auf Reis (Oryza sativa)

a) Reispflanzen der Sorte "Calora" oder "S6" werden nach 3-wöchiger Anzucht im Gewächshaus mit der Prüfsubstanz in Form einer Spritzbrühe (0,02 % Aktivsubstanz) besprüht. Nach eintägigem Antrocken dieses Spritzbelags werden die Pflanzen in einem Klimaraum bei 24°C und 75-85 % relativer Luftfeuchtigkeit aufgestellt und infiziert. Die Infektion erfolgt, indem die Blattspitzen mit einer Schere, die zuvor in eine Suspension von Xanthomonas oryzae eingetaucht worden war, abgeschnitten werden. Nach 10-tägiger Inkubation werden die angeschnittenen Blätter bei Befall welk, rollen sich ein und werden nekrotisch. Das Ausmass dieser Krankheitssymptome dient zur Beurteilung der residual Wirksamkeit der Prüfsubstanz.

b) Reispflanzen der Sorte "Calora" oder "S6" werden nach 3-wöchiger Anzucht im Gewächshaus mit einer Suspension der Prüfsubstanz begossen (0,006 % Aktivsubstanz bezogen auf das Erdvolumen). Drei Tage nach dieser Behandlung werden die Pflanzen in einem Klimaraum bei 24°C und 75-85 % relativer Luftfeuchtigkeit aufgestellt und infiziert. Die Infektion erfolgt, indem die Blattspitzen mit einer Schere, die zuvor in eine Suspension von Xanthomonas oryzae eingetaucht worden war, abgeschnitten werden. Nach 10-tägiger Inkubation werden die angeschnittenen Blätter bei Befall welk, rollen sich ein und werden nekrotisch. Das Ausmass dieser Krankheitssymptome dient zur Beurteilung der systemischen Wirksamkeit der Prüfsubstanz.

Verbindungen aus den Tabellen 1 bis 4 zeigten eine gute Schutzwirkung gegen Xanthomonas oryzae.

## Patentansprüche

## Patentansprüche für folgende Vertragsstaaten : AT, BE, CH, LI, DE, DK, FR, GB, GR, IT, LU, NL, SE

**1.** Verbindungen der Formel I

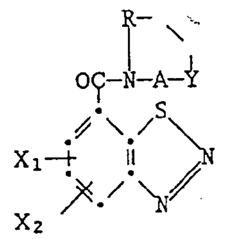

(I)

in welcher bedeuten:
$X_1$ und $X_2$ unabhängig voneinander Wasserstoff oder 1 bis 3 Halogen;
A Sulfonyl oder Carbonyl;
Y Phenyl oder mit $C_1$-$C_2$-Alkyl, $C_1$-$C_2$-Alkoxy, Halogen,- $C_1$-$C_2$-Haloalkyl, $C_1$-$C_2$-Alkoxycarbonyl, $C_1$-$C_3$-Alkanoyloxymethyl, Cyano und/oder Nitro substituiertes Phenyl;
R Wasserstoff, $C_1$-$C_4$-Alkyl, $C_1$-$C_4$-Haloalkyl, $C_3$-$C_5$-Alkenyl oder $C_3$-$C_5$-Alkinyl;
und in welcher ferner die Gruppe

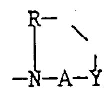

darstellt:

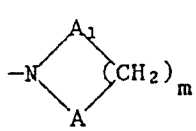 , 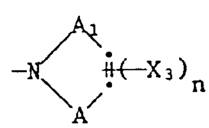 oder 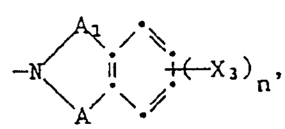

worin bedeuten:
$A_1$ Methylen, Carbonyl oder Sulfonyl;
$X_3$ Wasserstoff, Methyl oder Halogen;
m 2, 3 oder 4; und
n Null, 1 oder 2.

**2.** Verbindungen der Formel I gemäss Anspruch 1, worin bedeuten:
$X_1$ und $X_2$ unabhängig voneinander Wasserstoff oder 1 bis 3 Fluor;
A Sulfonyl oder Carbonyl;
Y mit Methyl, Methoxy, Halogen, Trifluormethyl, der $COOCH_3$-Gruppe, Cyano und/oder Nitro substituiertes Phenyl;
R Wasserstoff, Methyl, Ethyl, Trifluormethyl, Allyl oder Propargyl; und in welcher ferner die Gruppe

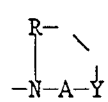

darstellt:

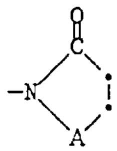

,

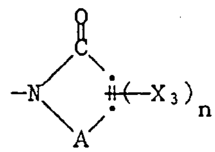

oder

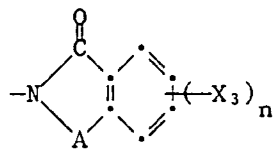

,

worin bedeuten:
A Sulfonyl oder Carbonyl;
$X_3$ Methyl oder Halogen; und
n Null, 1 oder 2.

3. Verbindungen der Formel I gemäss Anspruch 1, worin bedeuten:
$X_1$ und $X_2$ unabhängig voneinander Wasserstoff oder Fluor;
A Sulfonyl oder Carbonyl;
Y mit Methyl, Methoxy, Halogen, Trifluormethyl und/oder der $COOCH_3$-Gruppe substituiertes Phenyl;
R Wasserstoff, Methyl, Ethyl, Trifluormethyl, Allyl oder Propargyl; und
in welcher ferner die Gruppe

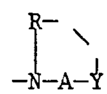

darstellt:

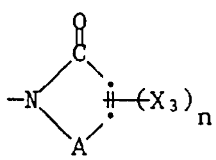

oder

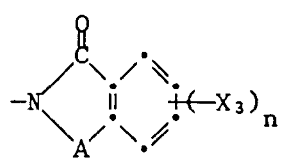

,

worin bedeuten:
A Sulfonyl oder Carbonyl;
$X_3$ Halogen; und
n Null oder 1.

4. Verbindungen der Formel I gemäss Anspruch 1, worin bedeuten:
$X_1$ und $X_2$ unabhängig voneinander Wasserstoff oder Fluor;
A Sulfonyl;
Y mit Methyl, Methoxy, Fluor, Chlor, Trifluormethyl und/oder der $COOCH_3$-Gruppe substituiertes Phenyl;
R Wasserstoff, Methyl, Trifluormethyl, Allyl oder Propargyl; und in welcher ferner die Gruppe

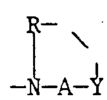

darstellt:

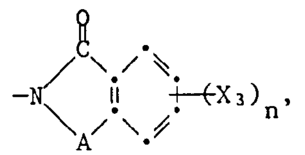

worin bedeuten:
A Sulfonyl;
$X_3$ Chlor; und
n Null oder 1.

**5.** Verbindungen der Formel I gemäss Anspruch 1, worin bedeuten:
$X_1$ und $X_2$ unabhängig voneinander Wasserstoff oder Fluor;
A Sulfonyl;
Y mit Methyl, Fluor, Chlor und/oder Trifluormethyl substituiertes Phenyl;
R Wasserstoff, Methyl, Allyl oder Propargyl;
und in welcher ferner für

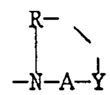

die Gruppe

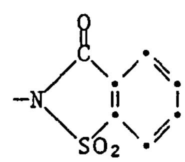

steht.

**6.** Eine Verbindung aus der Gruppe:
N-(Benz-1,2,3-thiadiazol-7-yl-carbonyl)-2-sulfobenzoesäureimid;
N-4-Chlorphenylsulfonyl-benz-1,2,3-thiadiazol-7-carbonsäureamid;
N-2-Fluorphenylsulfonyl-benz-1,2,3-thiadiazol-7-carbonsäureamid;
N-Allyl-N-phenylsulfonyl-benz-1,2,3-thiadiazol-7-carbonsäureamid;
N-Phenylsulfonyl-4-fluor-benz-1,2,3-thiadiazol-7-carbonsäureamid;
N-4-Methylphenylsulfonyl-5-fluor-benz-1,2,3-thiadiazol-7-carbonsäureamid;
N-Phenylsulfonyl-benz-1,2,3-thiadiazol-7-carbonsäureamid;
N-(5-Fluor-benz-1,2,3-thiadiazol-7-carbonyl)-2-sulfobenzoesäureimid.

**7.** Verfahren zur Herstellung von Verbindungen der Formel I in Anspruch 1, dadurch gekennzeichnet, dass man umsetzt:
eine Benzthiadiazolverbindung der Formel II

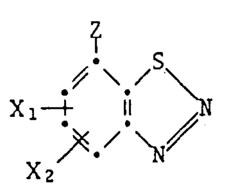

(II)

mit einer Aminoverbindung der Formel III

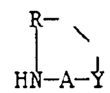

in Gegenwart einer Base gegebenenfalls mit einem Katalysator in inerten Lösungsmitteln bei Temperaturen von -10°C bis 200°C, wobei Z die Reste COOH, HalCO, $COOC_1$-$C_5$-Alkyl,

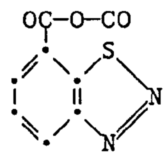

oder

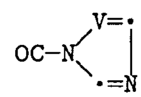

bedeutet, V CH oder N und Hal Halogen darstellen( und $X_1$, $X_2$, A, Y und R die unter der Formel I angegebenen Bedeutungen besitzen.

**8.** Verfahren zur Herstellung von Verbindungen der Formel I in Anspruch 1, dadurch gekennzeichnet, dass man eine Verbindung der Formel II

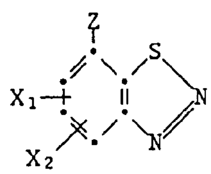

(II)

zunächst durch Reaktion mit einer Aminoverbindung der Formel IV

$H_2N$-R (IV)

in Gegenwart einer Base in inerten Lösungsmitteln in eine Amidverbindung der Formel V

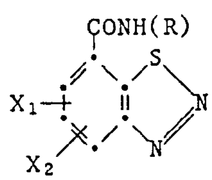

(V)

überführt und diese anschliessend mit einem Säurederivat der Formel VI

Z'-A-Y (VI)

in Gegenwart einer Base in inerten Lösungsmitteln bei Temperaturen von -10° bis 200°C umgesetzt, wobei Z' die Reste OH, Hal, $OC_1$-$C_5$-Alkyl, oder

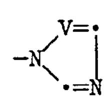

bedeutet,
oder der Rest Z'-A für

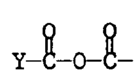

steht, V CH oder N und Hal Halogen darstellen und $X_1$, $X_2$, A, Y und R die unter der Formel I angegebenen Bedeutungen besitzen.

**9.** Mittel zum Schutz von Pflanzen gegen den Befall durch Mikroorganismen, dadurch gekennzeichnet, dass es neben üblichen Träger- und Hilfsstoffen als aktive Komponente mindestens eine Verbindung gemäss Anspruch 1 enthält.

**10.** Mittel gemäss Anspruch 9, dadurch gekennzeichnet, dass es als aktive Komponente mindestens eine Verbindung gemäss den Ansprüchen 2 bis 5 enthält.

**11.** Mittel gemäss Anspruch 9, dadurch gekennzeichnet, dass es als aktive Komponente eine Verbindung der Formel I gemäss Anspruch 6 enthält.

**12.** Verfahren zur Herstellung eines agrochemischen Mittels von Anspruch 9, dadurch gekennzeichnet, dass man mindestens eine gemäss Anspruch 1 definierte Verbindung mit geeigneten festen oder flüssigen Träger- und Hilfsstoffen innig vermischt.

**13.** Verwendung von Verbindungen gemäss Anspruch 1 zum Schutz von Pflanzen gegen den Befall durch phytopathogene Mikroorganismen.

**14.** Verwendung von Verbindungen gemäss einem der Ansprüche 2 bis 6 zum Schutz von Pflanzen gegen den Befall durch phytopathogene Mikroorganismen.

**15.** Verfahren zum Schutz von Pflanzen gegen den Befall durch phytopathogene Mikroorganismen, dadurch gekennzeichnet, dass man als Wirkstoff eine Verbindung gemäss Anspruch 1 auf die Pflanze oder deren Standort appliziert.

**16.** Verfahren zum Schutz von Pflanzen gegen den Befall durch phytopathogene Mikroorganismen, dadurch gekennzeichnet, dass man als Wirkstoff eine Verbindung gemäss einem der Ansprüche 2 bis 6 auf die Pflanze oder deren Standort appliziert.

**17.** Verfahren gemäss den Ansprüchen 15 und 16, dadurch gekennzeichnet, dass es sich bei den phytopathogenen Mikroorganismen um Pilzorganismen und/oder Bakterien handelt.

**Patentansprüche für folgenden Vertragsstaat : ES**

**1.** Verfahren zur Herstellung von Verbindungen der Formel I

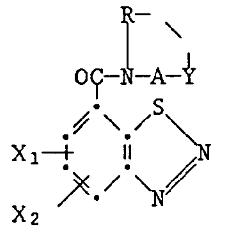

(I)

in welcher bedeuten:
$X_1$ und $X_2$ unabhängig voneinander Wasserstoff oder 1 bis 3 Halogen;
A Sulfonyl oder Carbonyl;
Y Phenyl oder mit $C_1$-$C_2$-Alkyl, $C_1$-$C_2$-Alkoxy, Halogen, $C_1$-$C_2$-Haloalkyl, $C_1$-$C_2$-Alkoxycarbonyl, $C_1$-$C_3$-Alkanoyloxymethyl, Cyano und/oder Nitro substituiertes Phenyl;
R Wasserstoff, $C_1$-$C_4$-Alkyl, $C_1$-$C_4$-Haloalkyl, $C_3$-$C_5$-Alkenyl oder $C_3$-$C_5$-Alkinyl;
und in welcher ferner die Gruppe

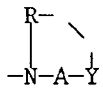

darstellt:

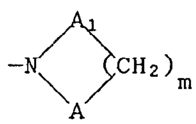 , 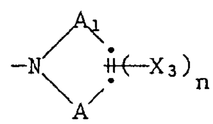 oder 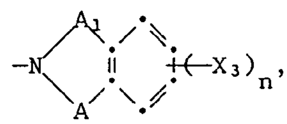

worin bedeuten:
$A_1$ Methylen, Carbonyl oder Sulfonyl;
$X_3$ Wasserstoff, Methyl oder Halogen;
m 2, 3 oder 4; und
n Null, 1 oder 2, dadurch gekennzeichnet, dass man umsetzt:
eine Benzthiadiazolverbindung der Formel II

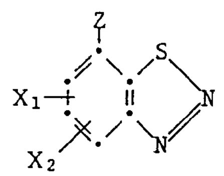 (II)

mit einer Aminoverbindung der Formel III

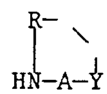

in Gegenwart einer Base gegebenenfalls mit einem Katalysator in inerten Lösungsmitteln bei Temperaturen von -10°C bis 200°C, wobei Z die Reste COOH, HalCO, $COOC_1$-$C_5$-Alkyl,

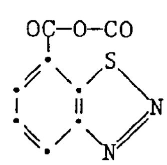 oder 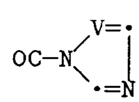

bedeutet, V CH oder N und Hal Halogen darstellen und $X_1$, $X_2$, A, Y und R die unter der Formel I angegebenen Bedeutungen besitzen.

2. Verfahren gemäss Anspruch 1, dadurch gekennzeichnet, dass Verbindungen hergestellt werden, worin bedeuten:
$X_1$ und $X_2$ unabhängig voneinander Wasserstoff oder 1 bis 3 Fluor;
A Sulfonyl oder Carbonyl;
Y mit Methyl, Methoxy, Halogen, Trifluormethyl, der $COOCH_3$-Gruppe, Cyano und/oder Nitro substituiertes Phenyl;
R Wasserstoff, Methyl, Ethyl, Trifluormethyl, Allyl oder Propargyl; und in welcher ferner die Gruppe

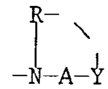

darstellt:

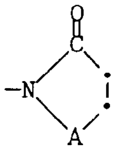 , 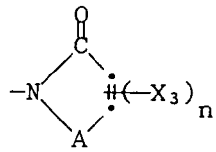 oder 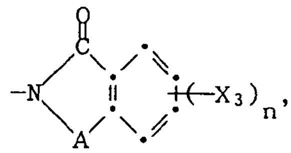

worin bedeuten:
A Sulfonyl oder Carbonyl;
$X_3$ Methyl oder Halogen; und
n Null, 1 oder 2.

3. Verfahren gemäss Anspruch 1, dadurch gekennzeichnet, dass Verbindungen hergestellt werden, worin bedeuten:
$X_1$ und $X_2$ unabhängig voneinander Wasserstoff oder Fluor;
A Sulfonyl oder Carbonyl;
Y mit Methyl, Methoxy, Halogen, Trifluormethyl und/oder der $COOCH_3$-Gruppe substituiertes Phenyl;
R Wasserstoff, Methyl, Ethyl, Trifluormethyl, Allyl oder Propargyl; und in welcher ferner die Gruppe

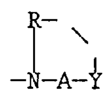

darstellt:

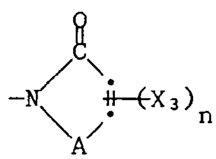 oder 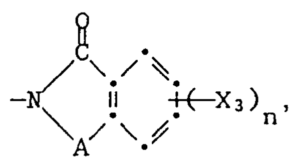

worin bedeuten:
A Sulfonyl oder Carbonyl;
$X_3$ Halogen; und
n Null oder 1.

4. Verfahren gemäss Anspruch 1, dadurch gekennzeichnet, dass Verbindungen hergestellt werden, worin bedeuten:
$X_1$ und $X_2$ unabhängig voneinander Wasserstoff oder Fluor;
A Sulfonyl;
Y mit Methyl, Methoxy, Fluor, Chlor, Trifluormethyl und/oder der $COOCH_3$-Gruppe substituiertes Phenyl;
R Wasserstoff, Methyl, Trifluormethyl, Allyl oder Propargyl; und in welcher ferner die Gruppe

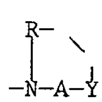

darstellt:

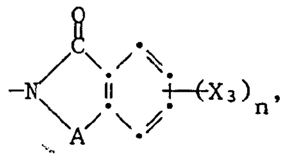

worin bedeuten:
A Sulfonyl;
$X_3$ Chlor; und
n Null oder 1.

5. Verfahren gemäss Anspruch 1, dadurch gekennzeichnet, dass Verbindungen hergestellt werden, worin bedeuten:
$X_1$ und $X_2$ unabhängig voneinander Wasserstoff oder Fluor;
A Sulfonyl;
Y mit Methyl, Fluor, Chlor und/oder Trifluormethyl substituiertes Phenyl;
R Wasserstoff, Methyl, Allyl oder Propargyl;
und in welcher ferner für

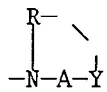

die Gruppe

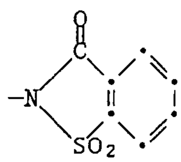

steht.

6. Verfahren gemäss Anspruch 1, dadurch gekennzeichnet, dass eine Verbindung hergestellt wird aus der Gruppe:
N-(Benz-1,2,3-thiadiazol-7-yl-carbonyl)-2-sulfobenzoesäureimid;
N-4-Chlorphenylsulfonyl-benz-1,2,3-thiadiazol-7-carbonsäureamid;
N-2-Fluorphenylsulfonyl-benz-1,2,3-thiadiazol-7-carbonsäureamid;
N-Allyl-N-phenylsulfonyl-benz-1,2,3-thiadiazol-7-carbonsäureamid;
N-Phenylsulfonyl-4-fluor-benz-1,2,3-thiadiazol-7-carbonsäureamid;
N-4-Methylphenylsulfonyl-5-fluor-benz-1,2,3-thiadiazol-7-carbonsäureamid;
N-Phenylsulfonyl-benz-1,2,3-thiadiazol-7-carbonsäureamid;
N-(5-Fluor-benz-1,2,3-thiadiazol-7-carbonyl)-2-sulfobenzoesäureimid.

7. Verfahren zur Herstellung von Verbindungen der Formel I in Anspruch 1, dadurch gekennzeichnet, dass man
eine Verbindung der Formel II

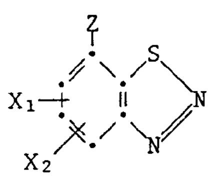

(II)

zunächst durch Reaktion mit einer Aminoverbindung der Formel IV

$H_2N\text{-}R$ (IV)

in Gegenwart einer Base in inerten Lösungsmitteln in eine Amidverbindung der Formel V

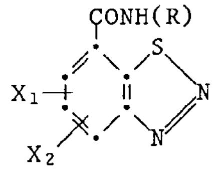

(V)

überführt und diese anschliessend mit einem Säurederivat der Formel VI

Z'-A-Y (VI)

in Gegenwart einer Base in inerten Lösungsmitteln bei Temperaturen von -10° bis 200°C umgesetzt, wobei Z' die Reste OH, Hal, $OC_1$-$C_5$-Alkyl, oder

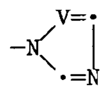

bedeutet,
oder der Rest Z'-A für

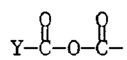

steht, V CH oder N und Hal Halogen darstellen und $X_1$, $X_2$, A, Y und R die unter der Formel I angegebenen Bedeutungen besitzen.

**8.** Mittel zum Schutz von Pflanzen gegen den Befall durch Mikroorganismen, dadurch gekennzeichnet, dass es neben üblichen Träger- und Hilfsstoffen als aktive Komponente mindestens eine Verbindung gemäss Anspruch 1 enthält.

**9.** Mittel gemäss Anspruch 8, dadurch gekennzeichnet, dass es als aktive Komponente mindestens eine Verbindung gemäss den Ansprüchen 2 bis 5 enthält.

**10.** Mittel gemäss Anspruch 8, dadurch gekennzeichnet, dass es als aktive Komponente eine Verbindung der Formel I gemäss Anspruch 6 enthält.

**11.** Verfahren zur Herstellung eines agrochemischen Mittels von Anspruch 8, dadurch gekennzeichnet, dass man mindestens eine gemäss Anspruch 1 definierte Verbindung mit geeigneten festen oder flüssigen Träger- und Hilfsstoffen innig vermischt.

**12.** Verwendung von Verbindungen gemäss Anspruch 1 zum Schutz von Pflanzen gegen den Befall durch phytopathogene Mikroorganismen.

**13.** Verwendung von Verbindungen gemäss einem der Ansprüche 2 bis 6 zum Schutz von Pflanzen gegen den Befall durch phytopathogene Mikroorganismen.

**14.** Verfahren zum Schutz von Pflanzen gegen den Befall durch phytopathogene Mikroorganismen, dadurch gekennzeichnet, dass man als Wirkstoff eine Verbindung gemäss Anspruch 1 auf die Pflanze oder deren Standort appliziert.

**15.** Verfahren zum Schutz von Pflanzen gegen den Befall durch phytopathogene Mikroorganismen, dadurch gekennzeichnet, dass man als Wirkstoff eine Verbindung gemäss einem der Ansprüche 2 bis 6 auf die Pflanze oder deren Standort appliziert.

**16.** Verfahren gemäss den Ansprüchen 14 und 15, dadurch gekennzeichnet, dass es sich bei den phytopathogenen Mikroorganismen um Pilzorganismen und/oder Bakterien handelt.

## Claims

### Claims for the following Contracting States : AT, BE, CH, LI, DE, DK, FR, GB, GR, IT, LU, NL, SE

1. A compound of formula I

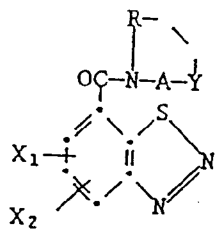

(I)

in which:
$X_1$ and $X_2$ independently of one another are hydrogen or from 1 to 3 halogen atoms;
A is sulfonyl or carbonyl;
Y is phenyl or phenyl substituted by $C_1$-$C_2$alkyl, $C_1$-$C_2$-alkoxy, halogen, $C_1$-$C_2$haloalkyl, $C_1$-$C_2$alkoxycarbonyl, $C_1$-$C_3$alkanoyloxymethyl, cyano and/or by nitro;
R is hydrogen, $C_1$-$C_4$alkyl, $C_1$-$C_4$haloalkyl, $C_3$-$C_5$alkenyl or $C_3$-$C_5$alkynyl;
and in which, furthermore, the group

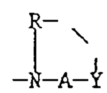

represents:

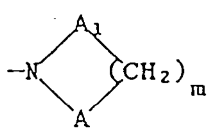 , 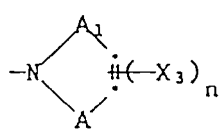 or 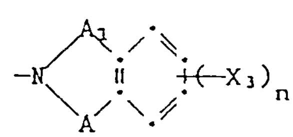 ,

wherein
$A_1$ is methylene, carbonyl or sulfonyl;
$X_3$ is hydrogen, methyl or halogen;
m is 2, 3 or 4; and
n is zero, 1 or 2.

2. A compound of formula I according to claim 1 in which:
$X_1$ and $X_2$ independently of one another are hydrogen or from 1 to 3 fluorine atoms;
A is sulfonyl or carbonyl;
Y is phenyl substituted by methyl, methoxy, halogen, trifluoromethyl, a $COOCH_3$ group, cyano and/or by nitro;
R is hydrogen, methyl, ethyl, trifluoromethyl, allyl or propargyl;
and in which, furthermore, the group

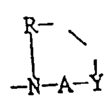

represents:

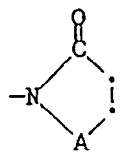 , 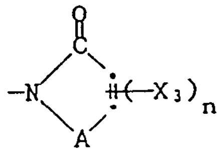 or 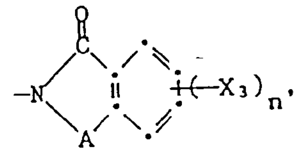

wherein
A is sulfonyl or carbonyl;
$X_3$ is methyl or halogen; and
n is zero, 1 or 2.

3. A compound of formula I according to claim 1 in which:
$X_1$ and $X_2$ independently of one another are hydrogen or fluorine;
A is sulfonyl or carbonyl;
Y is phenyl substituted by methyl, methoxy, halogen, trifluoromethyl and/or by a $COOCH_3$ group;
R is hydrogen, methyl, ethyl, trifluoromethyl, allyl or propargyl;
and in which, furthermore, the group

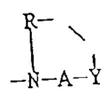

represents:

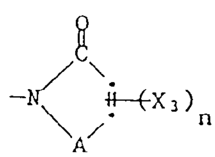 or 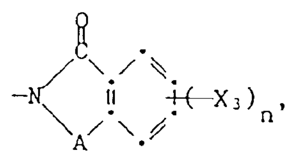

wherein
A is sulfonyl or carbonyl;
$X_3$ is halogen; and
n is zero or 1.

4. A compound of formula I according to claim 1 in which:
$X_1$ and $X_2$ independently of one another are hydrogen or fluorine;
A is sulfonyl;
Y is phenyl substituted by methyl, methoxy, fluorine, chlorine, trifluoromethyl and/or by a $COOCH_3$ group;
R is hydrogen, methyl, trifluoromethyl, allyl or propargyl;
and in which, furthermore, the group

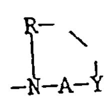

represents:

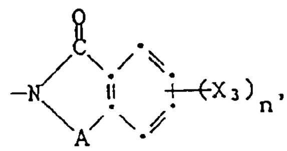

wherein
A is sulfonyl;
$X_3$ is chlorine; and
n is zero or 1.

5. A compound of formula I according to claim 1 in which:
$X_1$ and $X_2$ independently of one another are hydrogen or fluorine;
A is sulfonyl;
Y is phenyl substituted by methyl, fluorine, chlorine and/or by trifluoromethyl;
R is hydrogen, methyl, allyl or propargyl;
and in which, furthermore,

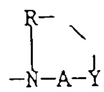

represents the group

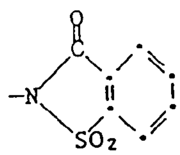

6. A compound from the group:
N-(benzo-1,2,3-thiadiazol-7-ylcarbonyl)-2-sulfobenzoic acid imide;
N-4-chlorophenylsulfonylbenzo-1,2,3-thiadiazole-7-carboxylic acid amide;
N-2-fluorophenylsulfonylbenzo-1,2,3-thiadiazole-7-carboxylic acid amide;
N-allyl-N-phenylsulfonylbenzo-1,2,3-thiadiazole-7-carboxylic acid amide;
N-phenylsulfonyl-4-fluorobenzo-1,2,3-thiadiazole-7-carboxylic acid amide;
N-4-methylphenylsulfonyl-5-fluorobenzo-1,2,3-thiadiazole-7-carboxylic acid amide;
N-phenylsulfonylbenzo-1,2,3-thiadiazole-7-carboxylic acid amide;
N-(5-fluorobenzo-1,2,3-thiadiazole-7-carbonyl)-2-sulfobenzoic acid imide.

7. A process for the preparation of a compound of formula I in claim 1 which comprises reacting:
a benzothiadiazole compound of formula II

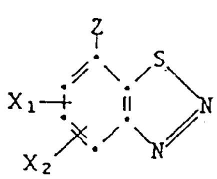

(II)

in the presence of a base and optionally with a catalyst, in an inert solvent at temperatures of from -10°C to 200°C, with an amino compound of formula III

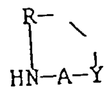

(III)

in which formulae Z represents the radical COOH, HalCO, $COOC_1$-$C_5$alkyl,

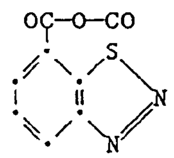 or 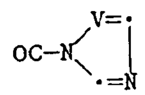 ,

V is CH or N and Hal is halogen, and $X_1$, $X_2$, A, Y and R are as defined for formula I.

**8.** A process for the preparation of a compound of formula I in claim 1 which comprises first of all converting a compound of formula II

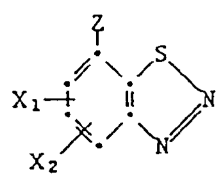

(II)

by reaction with an amino compound of formula IV

$H_2N$-R (IV)

in an inert solvent and in the presence of a base, into an amide compound of formula V

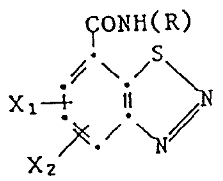

(V)

and then reacting this, in an inert solvent and in the presence of a base, at temperatures of from -10° to 200°C, with an acid derivative of formula VI

Z'-A-Y (VI)

in which formulae Z' represents the radical OH, Hal, $OC_1$-$C_5$alkyl or

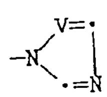 ,

or the radical Z'-A represents

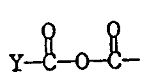 ,

V is CH or N and Hal is halogen, and $X_1$, $X_2$, A, Y and R are as defined for formula I.

**9.** A composition for protecting plants against attack by microorganisms that comprises as active component

at least one compound according to claim 1 together with customary carriers and adjuvants.

**10.** A composition according to claim 9 that comprises as active component at least one compound according to claims 2 to 5.

**11.** A composition according to claim 9 that comprises as active component a compound of formula I according to claim 6.

**12.** A process for the preparation of an agrochemical composition as claimed in claim 9 which comprises homogeneously mixing at least one compound defined in accordance with claim 1 with suitable solid or liquid carriers and adjuvants.

**13.** The use of a compound according to claim 1 for protecting plants against attack by phytopathogenic microorganisms.

**14.** The use of a compound according to any one of claims 2 to 6 for protecting plants against attack by phytopathogenic microorganisms.

**15.** A method of protecting plants against attack by phytopathogenic microorganisms which comprises applying as active ingredient to the plant or the locus thereof a compound according to claim 1.

**16.** A method of protecting plants against attack by phytopathogenic microorganisms which comprises applying as active ingredient to the plant or the locus thereof a compound according to any one of claims 2 to 6.

**17.** A method according to claims 15 and 16 wherein the phytopathogenic microorganisms are fungal organisms and/or bacteria.

**Claims for the following Contracting State : ES**

**1.** A process for the preparation of a compound of formula I

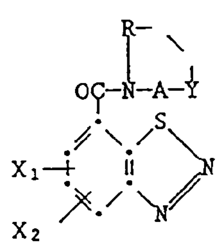

(I)

in which:
$X_1$ and $X_2$ independently of one another are hydrogen or from 1 to 3 halogen atoms;
A is sulfonyl or carbonyl;
Y is phenyl or phenyl substituted by $C_1$-$C_2$alkyl, $C_1$-$C_2$-alkoxy, halogen, $C_1$-$C_2$haloalkyl, $C_1$-$C_2$alkoxycarbonyl, $C_1$-$C_3$alkanoyloxymethyl, cyano and/or by nitro;
R is hydrogen, $C_1$-$C_4$alkyl, $C_1$-$C_4$haloalkyl, $C_3$-$C_5$alkenyl or $C_3$-$C_5$alkynyl;
and in which, furthermore, the group

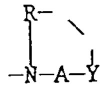

represents

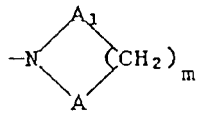 , 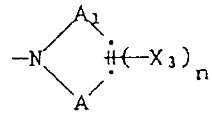 or 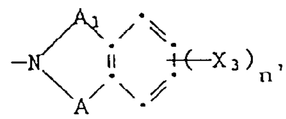

wherein
$A_1$ is methylene, carbonyl or sulfonyl;
$X_3$ is hydrogen, methyl or halogen;
m is 2, 3 or 4; and
n is zero, 1 or 2,
which comprises reacting:
a benzothiadiazole compound of formula II

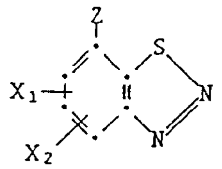

(II)

in the presence of a base and optionally with a catalyst, in an inert solvent at temperatures of from -10°C to 200°C, with an amino compound of formula III

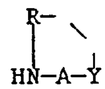

in which formulae Z represents the radical COOH, HalCO, $COOC_1$-$C_5$alkyl,

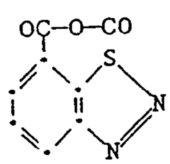 or 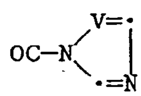 ,

V is CH or N and Hal is halogen, and $X_1$, $X_2$, A, Y and R are as defined for formula I.

2. A process according to claim 1, which comprises preparing a compound in which:
$X_1$ and $X_2$ independently of one another are hydrogen or from 1 to 3 fluorine atoms;
A is sulfonyl or carbonyl;
Y is phenyl substituted by methyl, methoxy, halogen, trifluoromethyl, a $COOCH_3$ group, cyano and/or by nitro;
R is hydrogen, methyl, ethyl, trifluoromethyl, allyl or propargyl;
and in which, furthermore, the group

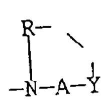

represents:

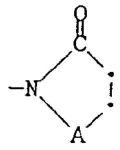 , 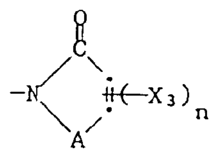 or 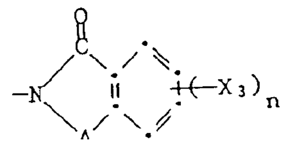,

wherein
A is sulfonyl or carbonyl;
$X_3$ is methyl or halogen; and
n is zero, 1 or 2.

**3.** A process according to claim 1, which comprises preparing a compound in which:
$X_1$ and $X_2$ independently of one another are hydrogen or fluorine;
A is sulfonyl or carbonyl;
Y is phenyl substituted by methyl, methoxy, halogen, trifluoromethyl and/or by a $COOCH_3$ group;
R is hydrogen, methyl, ethyl, trifluoromethyl, allyl or propargyl;
and in which, furthermore, the group

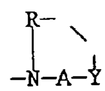

represents:

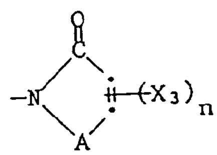 or 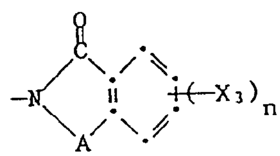,

wherein
A is sulfonyl or carbonyl;
$X_3$ is halogen; and
n is zero or 1.

**4.** A process according to claim 1, which comprises preparing a compound in which:
$X_1$ and $X_2$ independently of one another are hydrogen or fluorine;
A is sulfonyl;
Y is phenyl substituted by methyl, methoxy, fluorine, chlorine, trifluoromethyl and/or by a $COOCH_3$ group;
R is hydrogen, methyl, trifluoromethyl, allyl or propargyl;
and in which, furthermore, the group

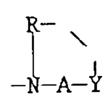

represents:

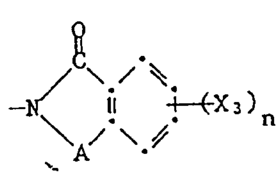,

wherein

A is sulfonyl;
$X_3$ is chlorine; and
n is zero or 1.

5. A process according to claim 1, which comprises preparing a compound in which:
$X_1$ and $X_2$ independently of one another are hydrogen or fluorine;
A is sulfonyl;
Y is phenyl substituted by methyl, fluorine, chlorine and/or by trifluoromethyl;
R is hydrogen, methyl, allyl or propargyl;
and in which, furthermore,

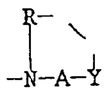

represents the group

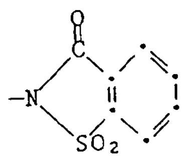

6. A process according to claim 1, which comprises preparing a compound from the group:
N-(benzo-1,2,3-thiadiazol-7-ylcarbonyl)-2-sulfobenzoic acid imide;
N-4-chlorophenylsulfonylbenzo-1,2,3-thiadiazole-7-carboxylic acid amide;
N-2-fluorophenylsulfonylbenzo-1,2,3-thiadiazole-7-carboxylic acid amide;
N-allyl-N-phenylsulfonylbenzo-1,2,3-thiadiazole-7-carboxylic acid amide;
N-phenylsulfonyl-4-fluorobenzo-1,2,3-thiadiazole-7-carboxylic acid amide;
N-4-methylphenylsulfonyl-5-fluorobenzo-1,2,3-thiadiazole-7-carboxylic acid amide;
N-phenylsulfonylbenzo-1,2,3-thiadiazole-7-carboxylic acid amide;
N-(5-fluorobenzo-1,2,3-thiadiazole-7-carbonyl)-2-sulfobenzoic acid imide.

7. A process for the preparation of a compound of formula I in claim 1 which comprises first of all converting a compound of formula II

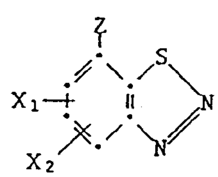

(II)

by reaction with an amino compound of formula IV

$$H_2N\text{-}R \qquad (IV)$$

in an inert solvent and in the presence of a base, into an amide compound of formula V

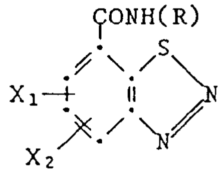

(V)

and then reacting this, in an inert solvent and in the presence of a base, at temperatures of from -10° to 200°C, with an acid derivative of formula VI

Z'-A-Y (VI)

in which formulae Z' represents the radical OH, Hal, $OC_1$-$C_5$alkyl or

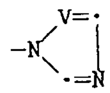,

or the radical Z'-A represents

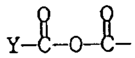,

V is CH or N and Hal is halogen, and $X_1$, $X_2$, A, Y and R are as defined for formula I.

8. A composition for protecting plants against attack by microorganisms that comprises as active component at least one compound according to claim 1 together with customary carriers and adjuvants.

9. A composition according to claim 8 that comprises as active component at least one compound according to claims 2 to 5.

10. A composition according to claim 8 that comprises as active component a compound of formula I according to claim 6.

11. A process for the preparation of an agrochemical composition as claimed in claim 8 which comprises homogeneously mixing at least one compound defined in accordance with claim 1 with suitable solid or liquid carriers and adjuvants.

12. The use of a compound according to claim 1 for protecting plants against attack by phytopathogenic microorganisms.

13. The use of a compound according to any one of claims 2 to 6 for protecting plants against attack by phytopathogenic microorganisms.

14. A method of protecting plants against attack by phytopathogenic microorganisms which comprises applying as active ingredient to the plant or the locus thereof a compound according to claim 1.

15. A method of protecting plants against attack by phytopathogenic microorganisms which comprises applying as active ingredient to the plant or the locus thereof a compound according to any one of claims 2 to 6.

16. A method according to claims 14 and 15 wherein the phytopathogenic microorganisms are fungal organisms and/or bacteria.

## Revendications

### Revendications pour les Etats contractants suivants : AT, BE, CH, LI, DE, DK, FR, GB, GR, IT, LU, NL, SE

1. Composés de formule I

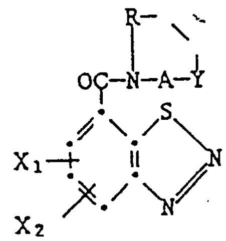

(I)

dans laquelle

| | |
|---|---|
| $X_1$ et $X_2$ | représentent indépendamment l'un de l'autre l'hydrogène ou 1 à 3 halogènes; |
| A | le sulfonyle ou le carbonyle; |
| Y | le phényle ou le phényle substitué par un alkyle en $C_1$-$C_2$, un alcoxy en $C_1$-$C_2$, un halogène, un haloalkyle en $C_1$-$C_2$, un alcoxy en $C_1$-$C_2$ carbonyle, un alcanoyle en $C_1$-$C_3$ oxyméthyle, le cyano et/ou le nitro; |
| R | l'hydrogène, un alkyle en $C_1$-$C_4$, un haloalkyle en $C_1$-$C_4$, un alcényle en $C_3$-$C_5$ ou un alcynyle en $C_3$-$C_5$; |

et dans laquelle, en outre, le groupe

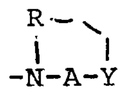

représente

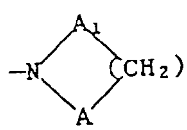 , 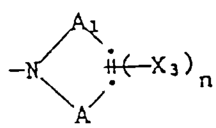 ou 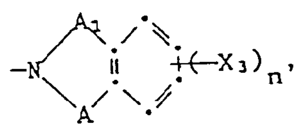

où

| | |
|---|---|
| $A_1$ | représente le méthylène, le carbonyle ou le sulfonyle; |
| $X_3$ | l'hydrogène, le méthyle ou un halogène; |
| m | est 2, 3 ou 4 et |
| n | est 0, 1 ou 2. |

**2.** Composés de formule I selon la revendication 1 où

| | |
|---|---|
| $X_1$ et $X_2$ | représentent indépendamment l'un de l'autre l'hydrogène ou 1 à 3 fluor ; |
| A | le sulfonyle ou le carbonyle; |
| Y | le phényle substitué par le méthyle, le méthoxy, un halogène, le trifluorométhyle, le groupe $COOCH_3$, le cyano et/ou le nitro; |
| R | l'hydrogène,le méthyle, l'éthyle, le trifluorométhyle, l'allyle ou le propargyle et dans laquelle, en outre, le groupe |

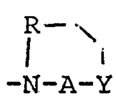

représente

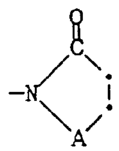 , 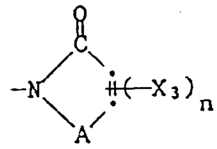 ou 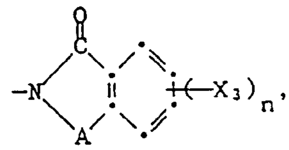

où

A représente le sulfonyle ou le carbonyle;

$X_3$ le méthyle ou un halogène et

n est 0, 1 ou 2.

**3.** Composés de formule I selon la revendication 1 où

$X_1$ et $X_2$ représentent indépendamment l'un de l'autre l'hydrogène ou le fluor;

A le sulfonyle ou le carbonyle;

Y le phényle substitué par le méthyle, le méthoxy, un halogène, le trifluorométhyle et/ou le groupe $COOCH_3$;

R l'hydrogène, le méthyle, l'éthyle, le trifluorométhyle, l'allyle ou le propargyle et dans laquelle, en outre, le groupe

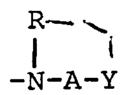

représente

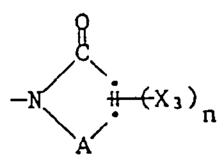 ou 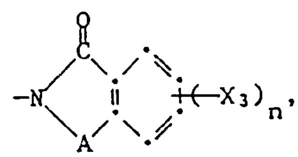

où

A représente le sulfonyle ou le carbonyle;

$X_3$ un halogène;

n est 0 ou 1.

**4.** Composés de formule I selon la revendication 1 où

$X_1$ et $X_2$ représentent indépendamment l'un de l'autre l'hydrogène ou le fluor;

A le sulfonyle;

Y le phényle substitué par le méthyle, le méthoxy, le fluor, le chlore, le trifluorométhyle et/ou le groupe $COOCH_3$;

R l'hydrogène, le méthyle, le trifluorométhyle, l'allyle ou le propargyle et dans laquelle, en outre, le groupe

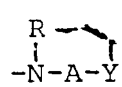

représente

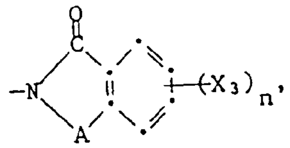

où
A représente le sulfonyle;
$X_3$ le chlore et
n est égal à 0 ou 1.

5. Composés de formule I selon la revendication 1 où
$X_1$ et $X_2$ représentent indépendamment l'un de l'autre l'hydrogène ou le fluor;
A le sulfonyle;
Y le phényle substitué par le méthyle, le fluor, le chlore et/ou le trifluorométhyle;
R l'hydrogène, le méthyle, l'allyle ou le propargyle;
et dans laquelle, en outre,

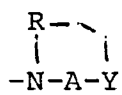

représente le groupe

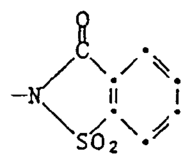

6. Un composé choisi dans le groupe :
l'imide d'acide N-(benz-1,2,3-thiadiazol-7-yl-carbonyl)-2-sulfobenzoïque;
l'amide d'acide N-4-chlorophénylsulfonyl-benz-1,2,3-thiadiazol-7-carboxylique;
l'amide d'acide N-2-fluorophénylsulfonyl-benz-1,2,3-thiadiazol-7-carboxylique;
l'amide d'acide N-allyl-N-phénylsulfonyl-benz-1,2,3-thiadiazol-7-carboxylique;
l'amide d'acide N-phénylsulfonyl-4-fluoro-benz-1,2,3-thiadiazol-7-carboxylique;
l'amide d'acide N-4-méthylphénylsulfonyl-5-fluoro-benz-1,2,3-thiadiazol-7-carboxylique;
l'amide d'acide N-phénylsulfonyl-benz-1,2,3-thiadiazol-7-carboxylique;
l'imide d'acide N-(5-fluoro-benz-1,2,3-thiadiazol-7-carbonyl)-2-sulfobenzoïque.

7. Procédé pour la préparation des composés de formule I selon la revendication 1, caractérisé en ce que l'on fait réagir :
un composé benzthiadiazol de formule II

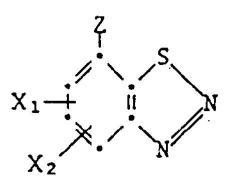

(II)

avec un composé amino de formule III

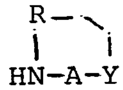

(III)

en présence d'une base éventuellement avec un catalyseur dans des solvants inertes à des températures allant de -10°C à 200°C, Z représentant les restes COOH, HalCO, COO-alkyle en $C_1$-$C_5$,

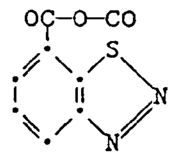

ou

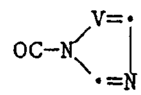

V représentan t CH ou N, Hal un halogène et $X_1$, $X_2$, A, Y et R ayant les significations données sous la formule I.

8. Procédé pour la préparation des composés de formule I selon la revendication 1, caractérisé en ce que l'on transforme tout d'abord un composé de formule II

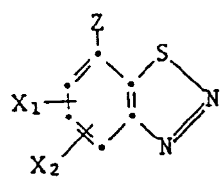

(II)

par réaction avec un composé amino de formule IV

$H_2N$-R (IV)

en présence d'une base dans des solvants inertes en un composé amide de formule V

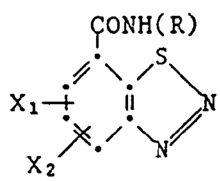

(V)

et en ce que l'on met celui-ci ensuite à réagir avec un dérivé d'acide de formule VI

Z'-A-Y (VI)

en présence d'une base dans des solvants inertes à des températures allant de -10°C à 200°C, Z' représentant les restes OH, Hal, O-alkyle en $C_1$-$C_5$ ou

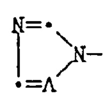

ou le reste Z'-A représentant

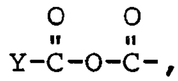

V représentant CH ou N et Hal représentant un halogène et $X_1$, $X_2$, A, Y et R ayant les significations données sous la formule I.

**9.** Agent pour la protection des plantes contre l'attaque par les microorganismes, caractérisé en ce qu'il contient, à côté des supports et adjuvants usuels, comme composant actif, au moins un composé selon la revendication 1.

**10.** Agent selon la. revendication 9, caractérisé en ce qu'il contient, comme composant actif, au moins un composé selon les revendications 2 à 5.

**11.** Agent selon la revendication 9, caractérisé en ce qu'il contient, comme composant actif, un composé de formule I selon la revendication 6.

**12.** Procédé pour la préparation d'un agent agrochimique selon la revendication 9, caractérisé en ce que l'on mélange intimement au moins un composé défini dans la revendication 1 avec des supports et adjuvants solides ou liquides appropriés.

**13.** Utilisation des composés selon la revendication 1 pour la protection des plantes contre l'attaque par les microorganismes phytopathogènes.

**14.** Utilisation des composés selon l'une des revendications 2 à 6 pour la protection des plantes contre l'attaque par les microorganismes phytopathogènes.

**15.** Procédé pour la protection des plantes contre l'attaque par les microorganismes phytopathogènes, caractérisé en ce qu'on l'on applique, comme substance active, un composé selon la revendication 1 sur la plante ou sur l'endroit où elle se trouve.

**16.** Procédé pour la protection des plantes contre l'attaque par les microorganismes phytopathogènes, caractérisé en ce que l'on applique, comme substance active, un composé selon l'une des revendications 2 à 6 sur la plante ou sur l'endroit où elle se trouve.

**17.** Procédé selon les revendications 15 et 16, caractérisé en ce que les microorganismes phytopathogènes sont des organismes provenant des champignons et/ou sont des bactéries.

**Revendications pour l'Etat contractant suivant : ES**

**1.** Procédé pour la préparation des composés de formule I

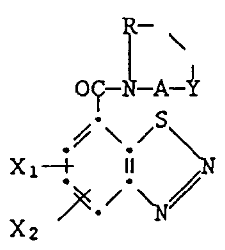

(I)

dans laquelle

| | |
|---|---|
| $X_1$ et $X_2$ | représentent indépendamment l'un de l'autre l'hydrogène ou 1 à 3 halogènes; |
| A | le sulfonyle ou le carbonyle; |
| Y | le phényle ou le phényle substitué par un alkyle en $C_1$-$C_2$, un alcoxy en $C_1$-$C_2$, un halogène, un haloalkyle en $C_1$-$C_2$, un alcoxy en $C_1$-$C_2$ carbonyle, un alcanoyle en $C_1$-$C_3$ oxyméthyle, le cyano et/ou le nitro; |

R l'hydrogène, un alkyle en $C_1$-$C_4$, un haloalkyle en $C_1$-$C_4$, un alcényle en $C_3$-$C_5$ ou un alcynyle en $C_3$-$C_5$;

et dans laquelle, en outre, le groupe

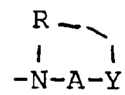

représente

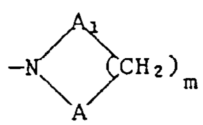 , 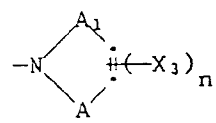 ou 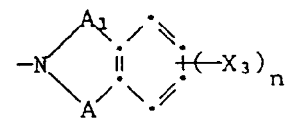 ,

où

- $A_1$ représente le méthylène, le carbonyle ou le sulfonyle;
- $X_3$ l'hydrogène, le méthyle ou un halogène;
- m est 2, 3 ou 4 et
- n est 0, 1 ou 2,

caractérisé en ce que l'on fait réagir :
un composé benzthiadiazol de formule II

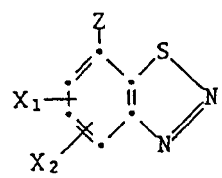

(II)

avec un composé amino de formule III

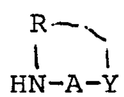

(III)

en présence d'une base éventuellement avec un catalyseur dans des solvants inertes à des températures allant de -10°C à 200°C, Z représentant les restes COOH, HalCO, COO-alkyle en $C_1$-$C_5$,

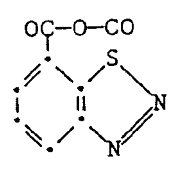 ou 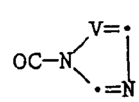

V représentant CH ou N, Hal un halogène et $X_1$, $X_2$, A, Y et R ayant les siqnifications données sous la formule I.

**2.** Procédé selon la revendication 1, caractérisé en ce que l'on prépare des composés dans lesquels

- $X_1$ et $X_2$ représentent indépendamment l'un de l'autre l'hydrogène ou 1 à 3 fluor ;
- A le sulfonyle ou le carbonyle;
- Y le phényle substitué par le méthyle, le méthoxy, un halogène, le trifluorométhyle, le groupe $COOCH_3$, le cyano et/ou le nitro;

R l'hydrogène, le méthyle, l'éthyle, le trifluorométhyle, l'allyle ou le propargyle et dans laquelle, en outre, le groupe

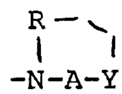

représente

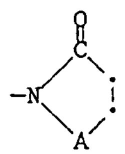 , 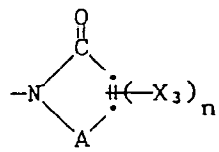 ou 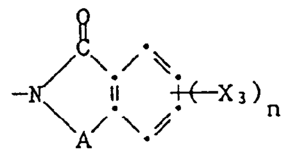

où
A représente le sulfonyle ou le carbonyle;
$X_3$ le méthyle ou un halogène et
n est 0, 1 ou 2.

**3.** Procédé selon la revendication 1, caractérisé en ce que l'on prépare des composés dans lesquels
$X_1$ et $X_2$ représentent indépendamment l'un de l'autre l'hydrogène ou le fluor;
A le sulfonyle ou le carbonyle;
Y le phényle substitué par le méthyle, le méthoxy, un halogène, le trifluorométhyle et/ou le groupe $COOCH_3$;
R l'hydrogène, le méthyle, l'éthyle, le trifluorométhyle, l'allyle ou le propargyle et dans laquelle, en outre, le groupe

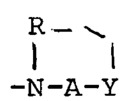

représente

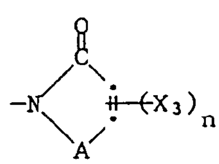 ou 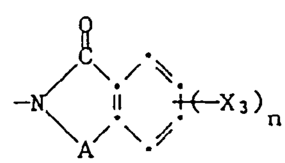

où
A représente le sulfonyle ou le carbonyle;
$X_3$ un halogène;
n est 0 ou 1.

**4.** Procédé selon la revendication 1, caractérisé en ce que l'on prépare des composés dans lesquels
$X_1$ et $X_2$ représentent indépendamment l'un de l'autre l'hydrogène ou le fluor;
A le sulfonyle;
Y le phényle substitué par le métyle, le méthoxy, le fluor, le chlore, le trifluorométhyle et/ou le groupe $COOCH_3$;
R l'hydrogène, le méthyle, le trifluorométhyle, l'allyle ou le propargyle et dans laquelle, en outre, le groupe

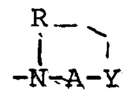

représente

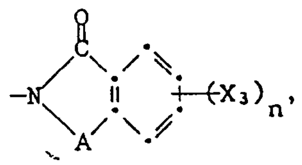

où

A représente le sulfonyle;

$X_3$ le chlore et

n est égal à 0 ou 1.

**5.** Procédé selon la revendication 1, caractérisé en ce que l'on prépare des composés dans lesquels

$X_1$ et $X_2$ représentent indépendamment l'un de l'autre l'hydrogène ou le fluor;

A le sulfonyle;

Y le phényle substitué par le méthyle, le fluor, le chlore et/ou le trifluorométhyle

R l'hydrogène, le méthyle, l'allyle ou le propargyle;

et dans laquelle, en outre,

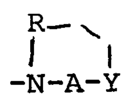

représente le groupe

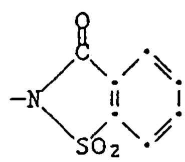

**6.** Procédé selon la revendication 1, caractérisé en ce que l'on prépare un composé choisi dans le groupe :
l'imide d'acide N-(benz-1,2,3-thiadiazol-7-yl-carbonyl)-2-sulfobenzoïque;
l'amide d'acide N-4-chlorophénylsulfonyl-benz-1,2,3-thiadiazol-7-carboxylique;
l'amide d'acide N-2-fluorophénylsulfonyl-benz-1,2,3-thiadiazol-7-carboxylique;
l'amide d'acide N-allyl-N-phénylsulfonyl-benz-1,2,3-thiadiazol-7-carboxylique;
l'amide d'acide N-phénylsulfonyl-4-fluoro-benz-1,2,3-thiadiazol-7-carboxylique;
l'amide d'acide N-4-méthylphénylsulfonyl-5-fluoro-benz-1,2,3-thiadiazol-7-carboxylique;
l'amide d'acide N-phénylsulfonyl-benz-1,2,3-thiadiazol-7-carboxylique;
l'imide d'acide N-(5-fluoro-benz-1,2,3-thiadiazol-7-carbonyl)-2-sulfobenzoïque.

**7.** Procédé pour la préparation des composés de formule I selon la revendication 1, caractérisé en ce que l'on transforme tout d'abord un composé de formule II

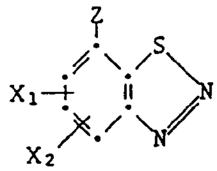
(II)

par réaction avec un composé amino de formule IV

$H_2N-R$ (IV)

en présence d'une base dans des solvants inertes en un composé amide de formule V

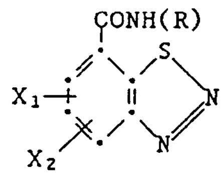
(V)

et en ce que l'on met celui-ci ensuite à réagir avec un dérivé d'acide de formule VI

Z'-A-Y (VI)

en présence d'une base dans des solvants inertes à des températures allant de -10°C à 200°C, Z' représentant les restes OH, Hal, O-alkyle en $C_1$-$C_5$ ou

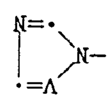

ou le reste Z'-A représentant

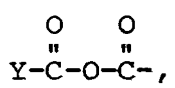

V représentant CH ou N et Hal représentant un halogène et $X_1$, $X_2$, A, Y et R ayant les significations données sous la formule I.

8. Agent pour la protection des plantes contre l'attaque par les microorganismes, caractérisé en ce qu'il contient, à côté des supports et adjuvants usuels, comme composant actif, au moins un composé selon la revendication 1.

9. Agent selon la revendication 8, caractérisé en ce qu'il contient, comme composant actif, au moins un composé selon les revendications 2 à 5.

10. Agent selon la revendication 8, caractérisé en ce qu'il contient, comme composant actif, un composé de formule I selon la revendication 6.

11. Procédé pour la préparation d'un agent agrochimique selon la revendication 8, caractérisé en ce que l'on mélange intimement au moins un composé défini dans la revendication 1 avec des supports et adjuvants solides ou liquides appropriés.

12. Utilisation des composés selon la revendication 1 pour la protection des plantes contre l'attaque parles microorganismes phytopathogènes.

13. Utilisation des composés selon l'une des revendications 2 à 6 pour la protection des plantes contre l'attaque par les microorganismes phytopathogènes.

**14.** Procédé pour la protection des plantes contre l'attaque par les microorganismes phytopathogènes, caractérisé en ce que l'on applique, comme substance active, un composé selon la revendication 1 sur la plante ou sur l'endroit où elle se trouve.

**15.** Procédé pour la protection des plantes contre l'attaque par les microorganismes phytopathogènes, caractérisé en ce que l'on applique, comme substance active, un composé selon l'une des revendications 2 à 6 sur la plante ou sur l'endroit où elle se trouve.

**16.** Procédé selon les revendications 14 et 15, caractérisé en ce que les microorganismes phytopathogènes sont des organismes provenant des champignons et/ou sont des bactéries.